(19) Europäisches Patentamt
European Patent Office
Office européen des brevets

(11) EP 1 923 410 A1

(12) # DEMANDE DE BREVET EUROPEEN

(43) Date de publication:
**21.05.2008 Bulletin 2008/21**

(51) Int Cl.:
***C08F 293/00*** *(2006.01)* ***C08F 291/00*** *(2006.01)*
***A61K 8/90*** *(2006.01)*

(21) Numéro de dépôt: **07120176.8**

(22) Date de dépôt: **07.11.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **16.11.2006 FR 0654942**

(71) Demandeur: **L'Oréal**
**75008 Paris (FR)**

(72) Inventeur: **Farcet, Céline**
**75012, PARIS (FR)**

(74) Mandataire: **Dodin, Catherine**
**L'OREAL - D.I.P.I.**
**25-29 Quai Aulagnier**
**92600 Asnières (FR)**

(54) **Nouveaux polymères séquencés, compositions les comprenant et procédé de traitement**

(57) L'invention concerne de nouveaux polymères séquencés comprenant de 35 à 100% en poids, par rapport au poids de la séquence les comprenant, de monomères siliconés choisis dans une liste donnée.

L'invention concerne également les compositions cosmétiques ou pharmaceutiques comprenant ces polymères, ainsi qu'un procédé de traitement cosmétique les employant, et un procédé de préparation de ces polymères.

EP 1 923 410 A1

## Description

La présente invention a trait à de nouveaux polymères de structure spécifique ainsi qu'aux compositions cosmétiques comprenant de tels polymères. L'invention concerne également un procédé de traitement cosmétique employant lesdits polymères.

Divers types de polymères sont conventionnellement utilisés dans les compositions cosmétiques en raison des diverses propriétés qu'ils peuvent leur apporter. Ils sont par exemple utilisés dans des compositions de maquillage ou de soin de la peau, des lèvres ou des phanères, telles que des vernis à ongles ou des compositions pour les cheveux.
Cependant, en utilisant au sein d'une même composition deux polymères incompatibles, c'est à dire non miscibles dans un même solvant, le formulateur est confronté, du fait de l'incompatibilité des polymères, à des problèmes de séparation de phases, voire de décantation, et de manière générale à l'obtention d'une composition non homogène. Ces problèmes ne pouvaient être jusqu'ici résolus que par la présence dans la composition d'un composé permettant de compatibiliser les polymères entre eux.
Pour pallier ce problème, il a été proposé, dans la demande EP1411069, des polymères de structure particulière, comprenant une première et au moins une deuxième séquence, incompatibles entre elles, et reliées par une séquence intermédiaire qui comprend au moins un monomère constitutif de chacune desdites deux séquences.
Cette demande décrit principalement de polymères séquencés préparés à partir de monomères de type acrylates et méthacrylates d'alkyle, notamment de méthyle, d'isobutyle, d'isobornyle, de trifluoroéthyle, ou d'acide (méth)acrylique.
Ces polymères sont généralement véhiculables, notamment solubles, dans des solvants organiques tels que les esters courts (acétate de butyle ou d'éthyle), les alcools courts tels que l'éthanol, ou les alcanes aliphatiques tels que l'isododécane. Ils sont toutefois assez difficilement véhiculables dans les solvants siliconés, usuellement employés en cosmétique.
Par ailleurs, les polymères selon EP1411069 permettent d'obtenir des films brillants, qui peuvent ne pas convenir dans certaines applications cosmétiques, dans le domaine des fonds de teints ou dans certaines compositions de soin de la peau.

La présente invention a pour but de proposer de nouveaux polymères, pouvant permettre l'obtention de films plus mats (moins brillants), ce qui permet d'éviter l'ajout usuel de charges dans la composition pour la matifier, lesdits polymères étant par ailleurs véhiculables à la fois dans des solvants carbonés et des solvants siliconés, ce qui n'était pas possible jusqu'alors.

Ainsi, un objet de la présente invention est un polymère séquencé comprenant au moins une première séquence et au moins une deuxième séquence, dans lequel au moins l'une des séquences comprend de 35 à 100% en poids, par rapport au poids de ladite séquence, d'au moins un monomère siliconé choisi parmi, seul ou en mélange, les monomères décrits ci-après.

L'invention a également pour objet une composition notamment cosmétique ou pharmaceutique comprenant, dans un milieu physiologiquement acceptable, un tel polymère.

Bien qu'il soit décrit dans EP1411069, d'une manière générale, la possibilité d'incorporer des monomères additionnels siliconés dans le polymère séquencé, il n'y est jamais décrit, ni même suggéré, la présence d'une quantité importante de monomères siliconés, et encore moins la possibilité d'obtenir les propriétés remarquables constatées avec les polymères selon l'invention.
Les polymères objet de la présente invention se différencient donc de ceux de l'art antérieur par la présence de monomères siliconés, en quantité importante, dans au moins une des séquences.

On a constaté que les polymères selon l'invention présentent une bonne solubilité dans les corps gras, qu'ils soient siliconés ou carbonés, que cela soit dans les huiles cosmétiques ou les solvants de type esters courts, solubilité qui peut varier et être ajustée selon la nature et/ou la quantité des monomères utilisés. Cette bonne liposolubilité peut faciliter leur mise en oeuvre ultérieure, notamment dans les compositions cosmétiques qui comprennent généralement une phase grasse.
De plus, les polymères selon l'invention permettent l'obtention de films mats, sans avoir à ajouter de charges.
Ces films sont moins cassants que ceux de l'art antérieur.
Ils sont également moins collants au dépôt et sont moins sensibles aux huiles que ceux ne comprenant pas de monomères siliconés. Ils présentent donc une meilleure résistance aux 'agressions' par des corps gras, tels que par exemple par l'huile alimentaire ou le sébum.
En outre, on a constaté que les polymères selon l'invention étaient de manière surprenante plus confortables que ceux de l'art antérieur ne comprenant pas ou peu de monomères siliconés. Le film se déforme peu ou pas du tout lors de son séchage, évitant ainsi les problèmes de tiraillement.
Enfin, la présence de monomères siliconés peut permettre d'obtenir des polymères présentant une viscosité plus basse que celle des polymères de l'art antérieur, ce qui permet de faciliter leur mise en oeuvre dans les compositions cosmétiques.

Les polymères selon la présente invention sont des polymères séquencés, comprenant au moins une première

séquence et au moins une deuxième séquence, ayant des températures de transition vitreuse (Tg) différentes, lesdites première et deuxième séquences étant avantageusement reliées entre elles par un segment intermédiaire comprenant au moins un monomère constitutif de la première séquence et au moins un monomère constitutif de la deuxième séquence.
On précise que dans ce qui précède et ce qui suit les termes "première" et "deuxième" séquences ne conditionnent nullement l'ordre desdites séquences (ou blocs) dans la structure du polymère.
Le polymère séquencé selon l'invention comprend donc au moins une première séquence et au moins une deuxième séquence, ces séquences étant avantageusement incompatibles l'une avec l'autre.
Par "séquences incompatibles l'une avec l'autre", on entend que le mélange formé du polymère correspondant à la première séquence et du polymère correspondant à la deuxième séquence, n'est pas miscible dans le solvant de polymérisation majoritaire en poids du polymère séquencé, à température ambiante (25°C) et pression atmosphérique ($10^5$ Pa), pour une teneur du mélange de polymères supérieure ou égale à 5 % en poids, par rapport au poids total du mélange (polymères et solvant), étant entendu que :

i) lesdits polymères sont présents dans le mélange en une teneur telle que le rapport pondéral respectif va de 10/90 à 90/10, et que
ii) chacun des polymères correspondant à la première et seconde séquences a une masse moléculaire moyenne (en poids ou en nombre) égale à celle du polymère séquencé +/- 15%.

Dans le cas d'un mélange de solvants de polymérisation, dans l'hypothèse de deux ou plusieurs solvants présents, ledit mélange de polymères est non miscible dans au moins l'un d'entre eux.
Bien entendu, dans le cas d'une polymérisation réalisée dans un solvant unique, ce dernier est le solvant majoritaire.
Lesdites première et deuxième séquences peuvent être avantageusement reliées entre elles par un segment intermédiaire comprenant au moins un monomère m1 constitutif de la première séquence et au moins un monomère m2 constitutif de la deuxième séquence.
Le segment intermédiaire forme une séquence (ou bloc). De préférence, m2 est différent de m1. Le segment ou séquence intermédiaire peut permettre de "compatibiliser" ces première et deuxième séquences.
Le polymère séquencé de la composition selon l'invention est avantageusement un polymère éthylénique séquencé, linéaire, ramifié ou greffé, de préférence formant un dépôt, et plus particulièrement filmogène.
Par polymère "éthylénique" , on entend un polymère obtenu par polymérisation de monomères comprenant une insaturation éthylénique.
Par polymère "séquencé", on entend un polymère comprenant au moins 2 séquences distinctes, de préférence au moins 3 séquences distinctes; de préférence diblocs ou triblocs.
Par polymère "formant un dépôt", on entend un polymère apte à former à lui seul ou en présence d'un agent auxiliaire, un dépôt adhérent sur un support, notamment sur les matières kératiniques.
Par polymère "filmogène", on entend un polymère apte à former à lui seul ou en présence d'un agent auxiliaire de filmification, un film continu et adhérent sur un support, notamment sur les matières kératiniques.
Chaque séquence, ou bloc, du polymère selon l'invention est issue d'un type de monomère ou de plusieurs types de monomères différents. Cela signifie que chaque séquence peut être constituée d'un homopolymère ou d'un copolymère, qui peut être statistique, alterné ou autre.
Avantageusement, lorsqu'il est présent, le segment intermédiaire comprenant au moins un monomère constitutif de la première séquence et au moins un monomère constitutif de la deuxième séquence du polymère est un polymère statistique. De préférence, la séquence intermédiaire est issue essentiellement de monomères constitutifs de la première séquence et de la deuxième séquence. Par "essentiellement", on entend au moins à 85%, de préférence au moins à 90%, mieux à 95% et encore mieux à 100%.
Selon l'invention, les première et deuxième séquences ont de préférence des températures de transition vitreuse différentes, avec un écart entre les températures de transition vitreuse des première et deuxième séquences généralement supérieur à 5°C, de préférence supérieur à 10°C, et mieux supérieur à 20°C.
Avantageusement, la séquence intermédiaire a une température de transition vitreuse Tg comprise entre les températures de transition vitreuse des première et deuxième séquences.
Les températures de transition vitreuse indiquées sont, sauf indication contraire, des Tg théoriques déterminées à partir des Tg théoriques des monomères constitutifs de chacune des séquences, que l'on peut trouver dans un manuel de référence tel que le Polymer Handbook, 4th éd. (Brandrup, Immergut, Grulke), 1999, John Wiley, selon la relation suivante, dite Loi de Fox :

$$\frac{1}{Tg} = \sum_i \left(\frac{\varpi i}{Tgi}\right)$$

wi étant la fraction massique du monomère i dans la séquence considérée et Tgi étant la température de transition vitreuse de l'homopolymère du monomère i (exprimée en Kelvin).

Le polymère selon l'invention se caractérise par le fait qu'au moins l'une de ses séquences comprend de 35 à 100% en poids, par rapport au poids de ladite séquence, d'au moins un monomère siliconé choisi parmi, seul ou en mélange, les monomères suivants :

- (i) les monomères éthyléniques dont le groupement ester contient des silanes et/ou des siloxanes, de formule :

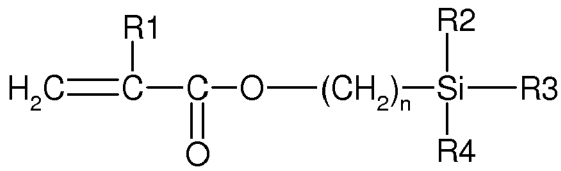

dans laquelle :

- R1= H ou méthyle,
- R2, R3, R4, identiques ou différents, représentent des groupes alkyles en C1-C6 ou le groupe $-OSi(R5)_3$ avec R5= méthyle ou éthyle; de préférence R2, R3 et/ou R4 représentent, indépendamment l'un de l'autre, $-OSi(Me)_3$ et/ou méthyle;
- n est un nombre entier de 1 à 10, de préférence égal à 1 ou 3.

- (ii) les macromonomères PDMS, ou polydiméthylsiloxanes à groupement terminal monoacryloyloxy ou monométhacryloyloxy, de formule suivante

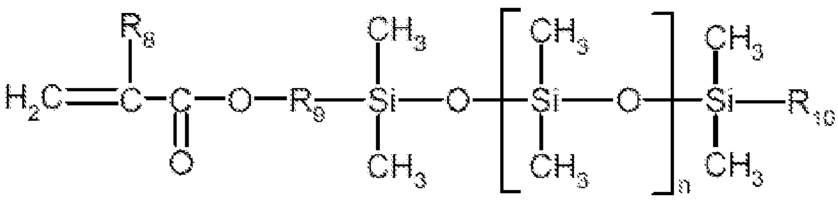

dans laquelle :

- R8 désigne un atome d'hydrogène ou un groupement méthyle; de préférence méthyle;
- R9 désigne un groupe hydrocarboné divalent, linéaire ou ramifié, de préférence linéaire, ayant de 1 à 10 atomes de carbone et contenant éventuellement une ou deux liaisons éther -O- ; de préférence éthylène, propylène ou butylène;
- R10 désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100.

- (iii) les monomères éthyléniques dont le groupement ester contient des carboxysilanes dendrimères de formule :

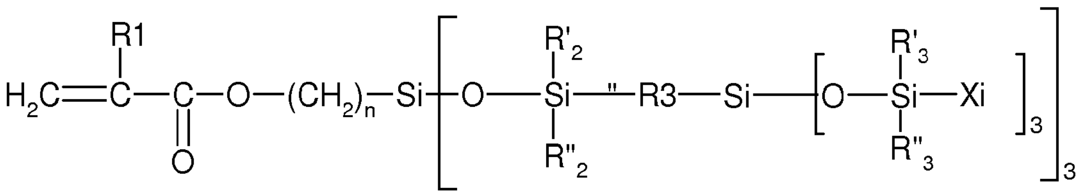

dans laquelle :

- R1= H ou méthyle;
- n est un nombre entier de 1 à 10, de préférence égal à 1, 2, 3 ou 4;
- R'2, R"2, R'3 et R"3, identiques ou différents, représentent un groupe alkyle en C1 à C10, de préférence méthyle ou éthyle;
- R3 est un groupe divalent alkylène en C2 à C10, de préférence en C2 ou C3;
- i est un entier compris entre 1 et 10, notamment i = 1, 2 ou 3; et
    - quand i = 2 à 10, X(i), identique ou différent, représente un groupe -R4-Si-[O-(R'3)(R"3)-X(i-1)]$_3$, avec R4, identique ou différent, représentant un groupe divalent alkylène en C2 à C10, de préférence en C2 ou C3;
    - et X(1) représente H ou un groupe alkyle en C1-C10, notamment méthyle ou éthyle;

et en particulier les monomères suivants, dans lesquels R1 = H ou méthyle :

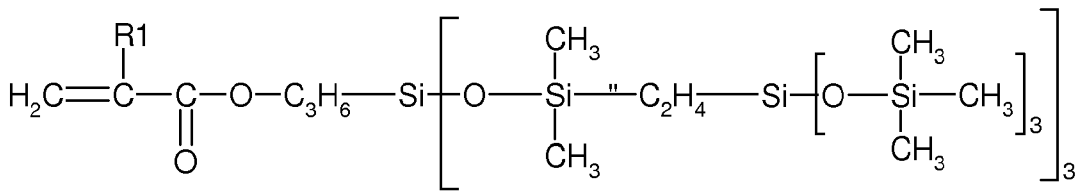

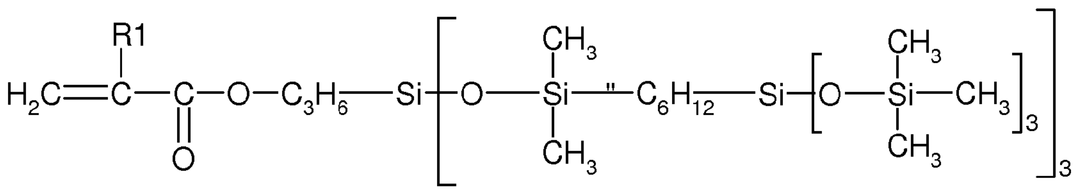

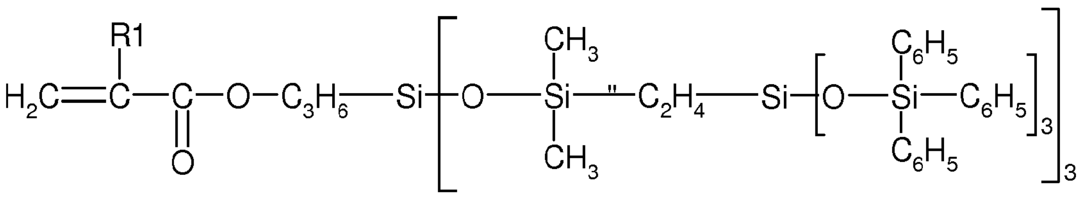

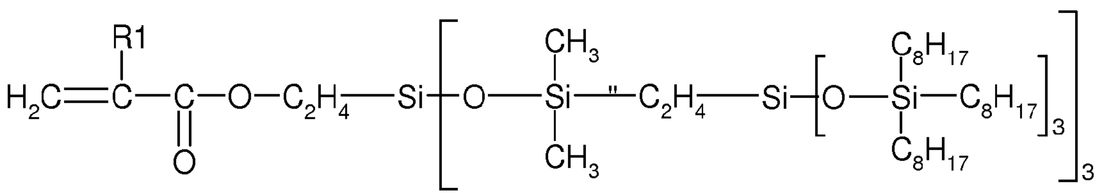

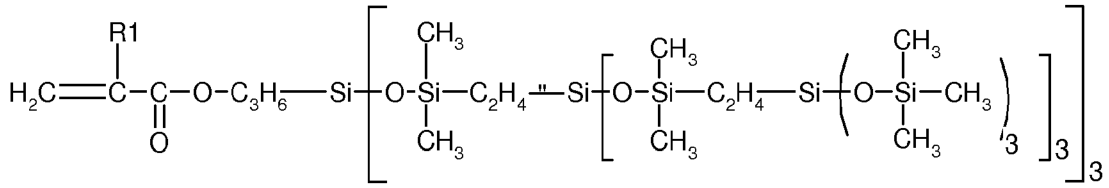

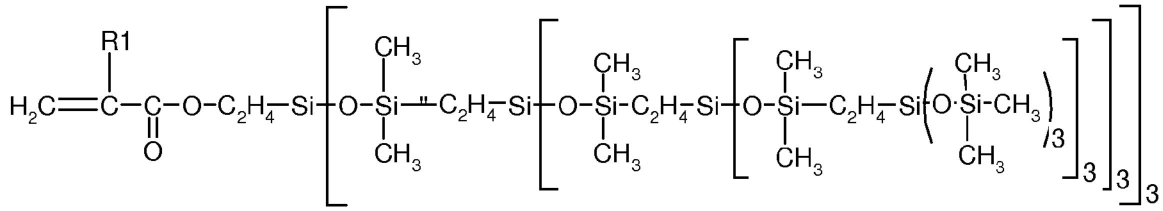

- (iv) les monomères éthyléniques de type POSS ou POS de structure :

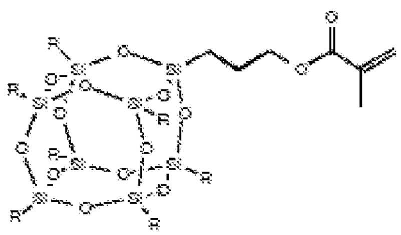

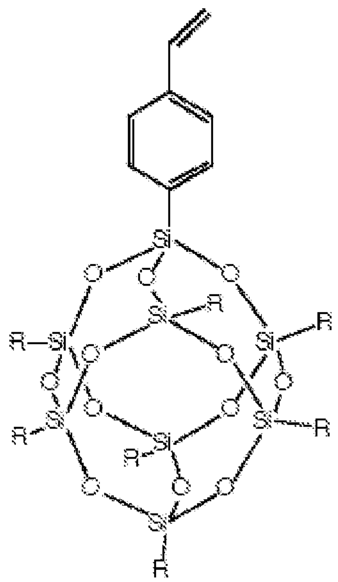

dans lesquelles R, identique ou différent, est un groupe alkyle linéaire en C1 à C10, de préférence méthyle, ou cyclique en C3 à C12, de préférence en C5.

On peut en particulier citer les monomères suivants : le (méth)acryloxypropyltris(trimethylsiloxy)silane, le (méth) acryloxypropylbis(trimethylsiloxy)méthylsilane, le (méth)acryloxyméthyltris(trimethylsiloxy)-silane, le (méth)acryloxyméthylbis(trimethylsiloxy)méthylsilane; le (méth)acryloxypropryltrimethoxysilane; et tout particulièrement les monomères suivants :

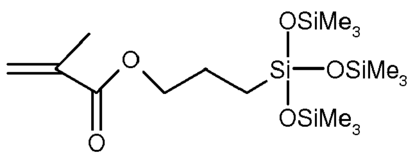

méthacryloxypropyltris(triméthylsiloxy)silane

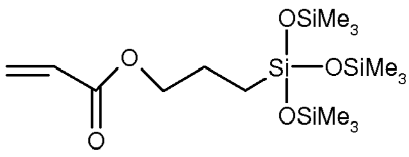

acryloxypropyltris(triméthylsiloxy)silane

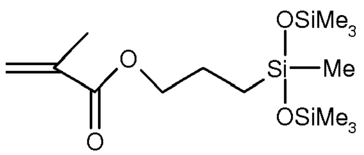

méthacryloxypropylbis(triméthylsiloxy)méthylsilane

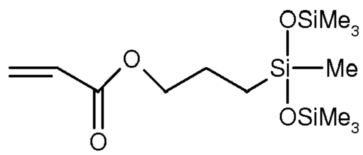

acryloxypropylbis(triméthylsiloxy)méthylsilane

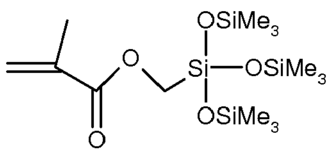

methacryloxymethyltris(trimethylsiloxy)silane

On peut également citer les monomères (méth)acrylique de type POSS (polyhedral oligomeric silsesquioxanes) et POS (polyhedral polymeric silicates), notamment d'Hybrid Plastics; et les monométhacryloyloxypropyl polydiméthylsiloxanes tels que ceux commercialisés sous la dénomination PS560-K6 par UCT (United Chemical Technologies Inc.) ou sous la dénomination MCR-M17 par Gelest Inc.

Les monomères siliconés ci-dessus représentent 35 à 100% en poids, notamment 50 à 99% en poids, voire 60 à 95% en poids, préférentiellement 70 à 90% en poids, du poids total de la séquence les comprenant.

De préférence, le ou les monomères siliconés tels que ci-dessus définis représentent 20 à 90% en poids, notamment 25 à 80% en poids, encore mieux 30 à 75% en poids, voire 35 à 70% en poids, du poids total du polymère. Ceci permet tout particulièrement d'obtenir un polymère soluble dans les phases grasses siliconées, notamment dans la D5 et/ou la phényltriméthicone, à 25 °C, 1 atm., à une teneur de 1 % en poids, avantageusement 10% en poids. Par soluble, on entend que le polymère forme une solution limpide, sans agrégats ni sédiments.

Les monomères siliconés sont de préférence présents, seuls ou en mélange, dans la deuxième séquence (formée en second lieu). Ils peuvent toutefois être présents dans la 1ère séquence, voire dans les 1ère et 2ème séquences, voire dans l'ensemble des séquences du polymère, y compris, ou non, la séquence intermédiaire.
De préférence, les monomères siliconés sont présents dans une séquence de Tg basse, c'est-à-dire une séquence dont la Tg est inférieure ou égale à 20°C, notamment inférieure ou égale à 0°C, voire inférieur ou égale à -10°C.

Par ailleurs, dans un mode de réalisation préféré, et afin notamment d'obtenir un polymère soluble dans les phases grasses carbonées, notamment dans les huiles telles que l'isododécane et/ou le Parléam, à 25°C, 1 atm., à une teneur de 1% en poids, avantageusement 10% en poids, le polymère peut comprendre 10 à 80% en poids, notamment 20 à 75% en poids, encore mieux 25 à 70% en poids, voire 30 à 65% en poids, d'un ou plusieurs monomères carbonés choisis dans une liste donnée.
Ces monomères carbonés choisis peuvent être présents dans la même séquence que les monomères siliconés, ou dans une séquence distincte; de préférence, ils sont présents dans une séquence distincte.

Les monomères carbonés choisis peuvent être choisis parmi, seuls ou en mélange, les monomères suivants :

- les méthacrylates de formule $CH_2 = C(CH_3)$-COOR1

dans laquelle R1 représente un groupe alkyle linéaire ou ramifié en C8-C22 tel que lauryle, béhényle ou stéaryle; ou

bien un groupe alkyle cyclique ayant 8 à 30 atomes de carbone, tel que isobornyle; ou encore R1 représente le groupe tertiobutyle;

- les acrylates de formule $CH_2$ = CH-COOR2

dans laquelle R2 représente un groupe alkyle linéaire ou ramifié en C8-C22 tel que lauryle, béhényle ou stéaryle ou 2-éthylhexyle; ou bien un groupe alkyle cyclique ayant 8 à 30 atomes de carbone, tel que isobornyle; ou encore R2 représente un groupe isobutyle ;

- les (méth)acrylamides de formule $CH_2$=C($CH_3$)-CONR3R4 ou $CH_2$=CH-CONR3R4,

dans lesquelles R3 représente un atome d'hydrogène ou un groupe alkyle linéaire ou ramifié en C1-C12, et R4 représente un groupe alkyle en C8 à C12 linéaire ou ramifié, tel qu'un groupe isooctyle, isononyle, undecyle;

- les di-n-alkylitaconates de formule $CH_2$=C($CH_2$-COO$(CH_2)_n$-$CH_3$)COO$(CH_2)_n$-$CH_3$, avec n étant un entier supérieur ou égal à 5, notamment de 6 à 13;
- les esters vinyliques de formule $CH_2$=CHOCOR5 où R5 représente un groupe alkyle en C8 à C22 linéaire ou ramifié;
- les éthers de vinyle de formule $CH_2$=CH-OR6 dans laquelle R6 représente un groupe alkyle linéaire ou ramifié, comprenant de 8 à 22 atomes de carbone;
- des macromonomères carbonés ayant un groupe terminal polymérisable;

On entend par "macromonomère ayant un groupe terminal polymérisable" tout oligomère comportant sur une seule de ses extrémités un groupe terminal polymérisable apte à réagir lors de la réaction de polymérisation avec des monomères éthyléniques. Le groupe polymérisable du macromonomère peut être avantageusement un groupe à insaturation éthylénique susceptible de se polymériser par voie radicalaire. Ledit groupe terminal polymérisable peut être en particulier un groupe vinyle ou (méth)acrylate (ou (méth)acryloxy), et de préférence un groupe (méth)acrylate. Par "macromonomère carboné" on entend un macromonomère non siliconé, et notamment un macromonomère oligomère obtenu par polymérisation de monomère(s) non siliconé(s) à insaturation éthylénique, et principalement par polymérisation de monomères acryliques et/ou vinyliques non acryliques.
Comme macromonomères carbonés ayant un groupe terminal polymérisable, on peut en particulier citer :

-(i) les homopolymères et les copolymères (méth)acrylate d'alkyle linéaire ou ramifié en C6-C22, de préférence en C8-C18, présentant un groupe terminal polymérisable choisi parmi les groupes vinyle ou (méth)acrylate parmi lesquels on peut citer en particulier les macromonomères de poly(acrylate d'éthyl-2 hexyle) à extrémité mono(méth) acrylate; les macromonomères de poly(acrylate de dodécyle) ou de poly(méthacrylate de dodécyle) à extrémité mono(méth)acrylate ; les macromonomères de poly(acrylate de stéaryle) ou de poly(méthacrylate de stéaryle) à extrémité mono(méth)acrylate. De tels macromonomères sont notamment décrits dans les brevets EP895467 et EP96459 et dans l'article Gillman , Polymer Letters, Vol 5, page 477-481 (1967).
On peut en particulier citer les macromonomères à base de poly(acrylate d'éthyl-2-hexyle) ou de poly(acrylate de dodécyle) à extrémité mono(méth) acrylate.
-(ii) les polyoléfines ayant un groupe terminal à insaturation éthylénique, en particulier ceux ayant un groupement terminal (méth)acrylate. Comme exemple de telles polyoléfines, on peut citer en particulier les macromonomères suivants, étant entendu qu'ils ont un groupe terminal (méth)acrylate : les macromonomères de polyéthylène, les macromonomères de polypropylène, les macromonomères de copolymère polyéthylène/polypropylène, les macromonomères de copolymère polyéthylène/polybutylène, les macromonomères de polyisobutylène; les macromonomères de polybutadiène; les macromonomères de polyisoprène; les macromonomères de polybutadiène; les macromonomères de poly(éthylène/butylène)-polyisoprène. De tels macromonomères sont en particulier décrits dans EP1347013 ou dans US5,625,005 qui mentionne des macromonomères éthylène/butylène et éthylène/propylène à groupement terminal réactif (méth)acrylate. On peut en particulier citer le méthacrylate de poly(éthylène/butylène), tel que celui commercialisé sous la dénomination Kraton Liquid L-1253 par Kraton Polymers.

Comme monomère carboné choisi particulièrement préféré, on peut citer, seuls ou en mélange, les méthacrylates de lauryle, de béhényle, de stéaryle, d'isobornyle, de tertiobutyle; les acrylates de lauryle, de béhényle, de 2-éthylhexyle, de stéaryle, d'isobornyle, d'isobutyle; les macromonomères à base de poly(acrylate d'éthyl-2-hexyle) ou de poly(acrylate de dodécyle) à extrémité mono(méth)acrylate; des macromonomères éthylène/butylène et éthylène/propylène à groupement terminal réactif (méth)acrylate.
Lorsque les monomères siliconés ne représentent pas 100% en poids de leur séquence, cette dernière comprend donc des monomères supplémentaires, qui peuvent être choisis parmi les monomères carbonés choisis et/ou les

monomères additionnels, et qui peuvent donc être présents à raison de 0,1% à 65% en poids, notamment 1% à 50% en poids, voire 5% à 40% en poids, préférentiellement 10 à 30% en poids, par rapport au poids total de la séquence.
La séquence comprenant ledit monomère siliconé peut donc être un homopolymère ne comprenant qu'un seul monomère siliconé; un copolymère comprenant plusieurs monomères siliconés décrits ci-dessus, ou un copolymère comprenant un ou plusieurs monomères siliconés décrits ci-dessus et un ou plusieurs monomères autres (carbonés choisis et/ou additionnels). Lorsque la séquence est un copolymère, ce dernier peut être, ainsi que cela a déjà été mentionné, statistique, alterné ou autre.
De la même façon, lorsque les monomères carbonés choisis ne représentent pas 100% en poids de leur séquence, cette dernière comprend donc des monomères siliconés et/ou additionnels, identiques ou différents des monomères additionnels de la séquence siliconée, qui peuvent être présents à raison de 0,1% à 65% en poids, notamment 1% à 50% en poids, voire 5% à 40% en poids, préférentiellement 10 à 30% en poids, par rapport au poids total de la séquence.
Dans ce cas, les monomères carbonés choisis peuvent de préférence représenter 35 à 99,9% en poids, notamment 50 à 99% en poids, voire 60 à 95% en poids, et encore mieux 70 à 90% en poids, par rapport au poids total de la séquence.
Les monomères additionnels peuvent être choisis parmi, seul ou en mélange, les monomères suivants :

-(i) les hydrocarbures éthyléniques ayant 2 à 10 carbones, tels que l'éthylène, l'isoprène, ou le butadiène ;

-(ii) les (méth)acrylates de formule $CH_2 = CHCOOR'_3$ ou $CH_2 = C(CH_3)COOR'_3$ dans lesquelles $R'_3$ représente :

- un groupe alkyle linéaire ou ramifié, de 1 à 7 atomes de carbone, notamment 4 à 7 dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle ou les atomes d'halogène (Cl, Br, I et F);
  notamment R'3 peut être un groupe méthyle, éthyle, propyle, n-butyle, isobutyle, tertiobutyle, hexyle; éthyl-2-perfluorohexyle; ou un groupe hydroxyalkyle en $C_{1-4}$ tel que 2-hydroxyéthyle, 2-hydroxybutyle et 2-hydroxypropyle; ou un groupe alcoxy ($C_{1-4}$) alkyle ($C_{1-4}$) tel que méthoxyéthyle, éthoxyéthyle et méthoxypropyle,
- un groupe cycloalkyle en C3-C7, tel que le groupe cyclohexyle,
- un groupe aryle en $C_3$ à $C_{20}$ tel que le groupe phényle,
- un groupe aralkyle en $C_4$ à $C_{30}$ (groupe alkyle en $C_1$ à $C_8$) tel que 2-phényl-éthyle, t-butylbenzyle ou benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle de 1 à 4 C), tel que furfurylméthyle ou tétrahydrofurfurylméthyle,
  lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle ou les atomes d'halogène (Cl, Br, I et F),
- $R'_3$ peut également être un groupe $-(C_2H_4O)_m-R''$, avec m = 5 à 150 et R'' = H ou alkyle de $C_1$ à $C_{30}$, par exemple -POE-méthyle ou -POE-béhényle;

ou encore le méthacrylate d'isobutyle ou l'acrylate de tertiobutyle;

-(iii) les (méth)acrylamides de formule :

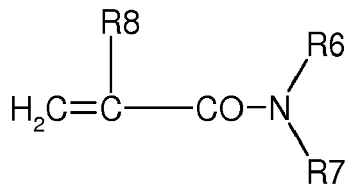

dans laquelle $R_8$ désigne H ou méthyle; et $R_7$ et $R_6$ identiques ou différents représentent :

- un atome d'hydrogène,
- un groupe alkyle linéaire ou ramifié, de 1 à 22 atomes de carbone, notamment 13 à 20, voire 14 à 18 atomes de carbone, dans lequel se trouve(nt) éventuellement intercalé(s) un ou plusieurs hétéroatomes choisis parmi

O, N, S et P; ledit groupe alkyle pouvant, en outre, être éventuellement substitué par un ou plusieurs substituants choisis parmi les groupes hydroxyle, les atomes d'halogène (Cl, Br, I et F); notamment R6 et/ou R7 peut être un groupe méthyle, éthyle, propyle, n-butyle, isobutyle, tertiobutyle, hexyle, éthylhexyle, octyle, lauryle, isooctyle, isodécyle, dodécyle, béhényle, stéaryle; éthyl-2-perfluorohexyle; ou un groupe hydroxyalkyle en $C_{1-4}$ tel que 2-hydroxyéthyle, 2-hydroxybutyle et 2-hydroxypropyle; ou un groupe alcoxy ($C_{1-4}$) alkyle ($C_{1-4}$) tel que méthoxyéthyle, éthoxyéthyle et méthoxypropyle,

- un groupe cycloalkyle en $C_3$ à $C_{12}$, tel que le groupe isobornyle, cyclohexyle, t-butylcyclohexyle;
- un groupe aryle en $C_3$ à $C_{20}$ tel que le groupe phényle,
- un groupe aralkyle en $C_4$ à $C_{30}$ (groupe alkyle en $C_1$ à $C_8$) tel que 2-phényl-éthyle, t-butylbenzyle ou benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4), tel que furfurylméthyle ou tétrahydrofurfurylméthyle,

lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F).
Des exemples de monomères (méth)acrylamide sont le (méth)acrylamide, le N-éthyl(méth)acrylamide, le N-butylacrylamide, le N-t-butylacrylamide, le N-isopropylacrylamide, le N,N-diméthyl(méth)acrylamide, le N,N-dibutylacrylamide, et le N(2-hydroxypropylméthacrylamide).

-(iv) les composés vinyliques de formules :

$$CH_2=CH-R_9,\ CH_2=CH-CH_2-R_9 \text{ ou } CH_2=C(CH_3)-CH_2-R_9$$

dans lesquelles $R_9$ est un groupe hydroxyle, halogène (Cl ou F), $NH_2$, $OR_{14}$ où $R_{14}$ représente un groupe phényle ou un groupe alkyle en $C_1$ à $C_7$; acétamide ($NHCOCH_3$); un groupe $OCOR_{15}$ où $R_{15}$ représente un groupe alkyle de 2 à 7 atomes de carbones, linéaire ou ramifié; ou un groupe choisi parmi :

- un groupe alkyle linéaire ou ramifié, de 1 à 22 atomes de carbone, notamment 4 à 20, voire 6 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl,Br,I et F),
- un groupe cycloalkyle en $C_3$ à $C_{12}$ tel que isobornyle, cyclohexyle,
- un groupe aryle en $C_3$ à $C_{20}$ tel que phényle,
- un groupe aralkyle en $C_4$ à $C_{30}$ (groupe alkyle en $C_1$ à $C_8$) tel que 2-phényléthyle; benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4), tel que furfurylméthyle ou tétrahydrofurfurylméthyle,

lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle les atomes d'halogène (Cl, Br, I et F);
Des exemples de monomères vinyliques sont le vinylcyclohexane, et le styrène. Des exemples d'esters vinyliques sont l'acétate de vinyle, le propionate de vinyle, le butyrate de vinyle. Parmi les éthers de vinyle, on peut citer le vinyl méthyl éther, le vinyl éthyl éther et le vinyl isobutyl éther.

-(v) les monomères à insaturation(s) éthylénique(s) comprenant au moins une fonction acide carboxylique, phosphorique ou sulfonique, ou anhydride, comme par exemple l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci;

-(vi) les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci.

Les sels peuvent être formés par neutralisation des groupes anioniques à l'aide d'une base minérale, telle que LiOH, NaOH, KOH, $Ca(OH)_2$, $NH_4OH$ ou $Zn(OH)_2$; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la diméthylamino-2-propanol. On peut encore citer la lysine ou la 3-(diméthylamino)-propylamine.

On peut également peut citer les sels d'acides minéraux, tels que l'acide sulfurique, l'acide chlorhydrique, l'acide bromhydrique, l'acide iodhydrique, l'acide phosphorique, l'acide borique. On peut aussi citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

On choisit plus particulièrement les monomères additionnels parmi, seuls ou en mélange l'acide (méth)acrylique et les (méth)acrylates d'alkyle en C1-C4, ou de cycloalkyle en C3-C7, et notamment le méthacrylate de méthyle, le méthacrylate d'isobutyle; l'acrylates de tertiobutyle, l'acide acrylique et l'acide méthacrylique.
De préférence, le ou les monomères additionnels tels que ci-dessus définis représentent 0 à 70% en poids, notamment 0,1 à 30% en poids, encore mieux 0,5 à 20% en poids, voire 1 à 10% en poids, du poids total du polymère.

De préférence, les monomères siliconés sont présents dans une séquence différente de celle dans laquelle les monomères carbonés choisis sont présents.
Le polymère selon l'invention peut en outre comprendre d'autres séquences, qui peuvent être de toute nature chimique.

De préférence, le polymère selon l'invention comprend une séquence de Tg supérieure ou égale à 20°C, par exemple comprise entre 20°C et 160°C, notamment comprise entre 30°C et 140°C, voire comprise entre 40°C et 120°C, préférentiellement comprise entre 50°C et 110°C, et qui est de préférence constituée des monomères carbonés.
Il comprend également de préférence une séquence dont la Tg est inférieure à 20°C, par exemple comprise entre -150°C et 20°C exclu, notamment comprise entre -100°C et 10°C, voire entre -85°C et 0°C, préférentiellement comprise entre -70°C et -5°C, et qui comprend de préférence les monomères siliconés.
De préférence, la/les séquences de Tg supérieure à 20°C représentent 50 à 90% en poids, notamment 60 à 80% en poids, voire 65 à 75% en poids, du poids total du polymère.

La séquence ayant une Tg supérieure ou égale à 20°C peut notamment comprendre, en totalité ou en partie, des monomères dont les homopolymères ont une Tg supérieure ou égale à 20°C. Elle peut également comprendre des monomères ayant une Tg en dehors de cette gamme. Ces monomères et leur concentration seront choisis de manière adéquate par l'homme du métier, notamment sur la base de la loi de Fox, pour obtenir une séquence de Tg souhaitée. Parmi ces monomères ayant une Tg supérieure ou égale à 20°C, on peut citer, seuls ou en mélange :

- les méthacrylates de formule : $CH_2 = C(CH_3)\text{-}COOR_1$ dans laquelle $R_1$ représente un groupe alkyle non substitué, linéaire ou ramifié, contenant de 1 à 4 atomes de carbone, tel qu'un groupe méthyle, éthyle, propyle ou isobutyle, ou bien $R_1$ représente un groupe cycloalkyle $C_4$ à $C_{12}$, notamment isobornyle;
- les acrylates de formule : $CH_2 = CH\text{-}COOR_2$ dans laquelle $R_2$ représente un groupe tertiobutyle ou un groupe cycloalkyle en $C_4$ à $C_{12}$ tel que un groupe isobornyle;
- les (méth)acrylamides de formule : $CH_2 = CR'\text{-}CO\text{-}NR^7R^8$ avec R' représentant H ou $CH_3$, et $R_7$ et $R_8$, identiques ou différents, représentent un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle; ou encore $R_7$ représente H et $R_8$ représente un groupement 1,1-diméthyl-3-oxobutyle,
- l'acide méthacrylique et l'acide acrylique.

Parmi les monomères dont les homopolymères ont une température de transition vitreuse Tg supérieure ou égale à 20°C plus particulièrement préférés, on peut citer le méthacrylate de méthyle, le méthacrylate d'éthyle, le méthacrylate d'isobutyle, le (méth)acrylate de tertio-butyle, l'acide (méth)acrylique, le (méth)acrylate d'isobornyle, le N-butylacrylamide, le N-t-butylacrylamide, le N-isopropylacrylamide, le N,N-diméthylacrylamide, le N,N-dibutylacrylamide et leurs mélanges.

La séquence ayant une Tg inférieure à 20°C peut notamment comprendre, en totalité ou en partie, des monomères dont les homopolymères ont une Tg inférieure à 20°C. Elle peut également comprendre des monomères ayant une Tg en dehors de cette gamme. Ces monomères et leur concentration seront choisis de manière adéquate par l'homme du métier, notamment sur la base de la loi de Fox, pour obtenir une séquence de Tg souhaitée. Parmi ces monomères ayant une Tg inférieure à 20°C, on peut citer, seuls ou en mélange :

- les acrylates de formule $CH_2 = CHCOOR_3$, $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs

hétéroatomes choisis parmi O, N, S,

- les méthacrylates de formule $CH_2 = C(CH_3)\text{-}COOR_4$, $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi O, N et S;
- les esters vinyliques de formule $R_5\text{-}CO\text{-}O\text{-}CH{=}CH_2$ où $R_5$ représente un groupe alkyle en $C_4$ à $C_{12}$ linéaire ou ramifié ;
- les ($C_4$-$C_{12}$alkyl)-vinyléthers, notamment le méthylvinyléther et l'éthylvinyléther;
- les N-($C_4$-$C_{12}$ alkyl)acrylamides, tels que le N-octylacrylamide,
- et leurs mélanges.

Parmi ces monomères de Tg inférieure à 20°C, on peut citer plus particulièrement l'acrylate de méthyle, l'acrylate d'éthyle, l'acrylate d'isobutyle, le (méth)acrylate d'éthyl-2-hexyle et leurs mélanges.

Dans un mode de réalisation préféré, le polymère selon l'invention comprend dans au moins une séquence, au moins un monomère choisi parmi les esters d'acide (méth)acrylique; éventuellement, il peut comprendre en outre au moins un deuxième monomère choisi parmi l'acide acrylique et l'acide méthacrylique, et leurs mélanges.

Préférentiellement, tous les monomères autres que les monomères siliconés sont choisis parmi les esters d'acide (méth) acrylique et l'acide (méth)acrylique.

La masse molaire moyenne en poids (Mw) du polymère selon l'invention est de préférence comprise entre 25 000 et 1 000 000, mieux entre 30 000 et 750 000, voire entre 40 000 et 500 000, et préférentiellement entre 50 000 et 250 000.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique et UV).

De préférence, l'indice de polydispersité du polymère selon l'invention est supérieur à 2, par exemple allant de 2 à 9, de préférence supérieur ou égal à 2,5, par exemple allant de 2,5 à 8, et mieux allant de 2,8 à 7. L'indice de polydispersité Ip du polymère est égal au rapport de la masse moyenne en poids Mw sur la masse moyenne en nombre Mn.

Le polymère selon l'invention peut être obtenu par polymérisation radicalaire en solution selon le procédé de préparation suivant :

- on peut introduire une partie du solvant de polymérisation dans un réacteur adapté, on chauffe jusqu'à atteindre la température adéquate pour la polymérisation (typiquement entre 60 et 120°C),
- une fois cette température atteinte, on peut ajouter les monomères constitutifs de la première séquence, en présence d'une partie de l'initiateur de polymérisation,
- au bout d'un temps T correspondant à un taux de conversion maximum de 90% de préférence, on peut introduire les monomères constitutifs de la deuxième séquence et l'autre partie de l'initiateur,
- on laisse réagir le mélange pendant un temps T' (allant notamment de 3 à 6 heures) au bout duquel le mélange est ramené à température ambiante (25°C), de manière à obtenir le polymère en solution dans le solvant de polymérisation.

Par solvant de polymérisation, on entend un solvant, ou un mélange de solvants, notamment choisi parmi l'acétate d'éthyle, l'acétate de butyle, les alcools en C1-C6 tels que l'isopropanol, l'éthanol, les alcanes aliphatiques tels que l'isododécane et leurs mélanges. De préférence, le solvant de polymérisation est un mélange d'acétate de butyle et d'isopropanol ou est l'isododécane, ou encore est la D5 (cyclopentadiméthylsiloxane).

De préférence, le polymère selon l'invention n'est pas hydrosoluble, c'est à dire que le polymère n'est pas soluble dans l'eau ou dans un mélange d'eau et de monoalcools linéaires ou ramifiés ayant de 2 à 5 atomes de carbone comme l'éthanol, l'isopropanol ou le n-propanol, sans modification de pH, à une teneur en matière active d'au moins 1% en poids, à température ambiante (25°C).

L'invention concerne également des compositions, notamment cosmétiques ou pharmaceutiques, comprenant au moins un polymère de structure spécifique tel que décrit ci-dessus.

De préférence, ledit polymère se trouve en solution dans le milieu physiologiquement, notamment cosmétiquement ou pharmaceutiquement, acceptable desdites compositions, en particulier en solution dans la phase grasse (ou une des phases grasses) desdites compositions, notamment dans une huile et/ou un solvant organique carboné et/ou siliconé.

Les polymères selon l'invention peuvent être présents, seuls ou en mélange, dans les compositions selon l'invention en une quantité de 0,01 à 50% en poids, de préférence 0,05 à 40% en poids, notamment 0,1 à 30% en poids, voire 0,5 à 20% en poids, et encore mieux 1 à 15% en poids, préférentiellement 1,5 à 12% en poids, par rapport au poids total de la composition.

Les compositions cosmétiques ou pharmaceutiques selon l'invention comprennent, outre lesdits polymères, un milieu physiologiquement acceptable, notamment cosmétiquement ou pharmaceutiquement, acceptable, c'est-à-dire un milieu compatible avec les matières kératiniques telles que la peau du visage ou du corps, les cheveux, les cils, les

sourcils et les ongles.

La composition peut ainsi comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le pentylène glycol, et les polyéthylène glycols, ou bien encore des éthers en $C_2$ et des aldéhydes en $C_2$-$C_4$ hydrophiles.

L'eau ou le mélange d'eau et de solvants organiques hydrophiles peut être présent dans la composition selon l'invention en une teneur allant de 0,1% à 99% en poids, par rapport au poids total de la composition, et de préférence de 10% à 80% en poids.

La composition peut également être anhydre.

La composition peut également comprendre une phase grasse qui peut comprendre des corps gras liquides à température ambiante (25°C en général) et/ou des corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.

Comme corps gras liquides à température ambiante, appelés souvent huiles, utilisables dans l'invention, on peut citer : les huiles hydrocarbonées d'origine animale telles que le perhydrosqualène; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyltrisiloxanes, les diphényl diméthicones, les phényl diméthicones, les polyméthylphényl siloxanes ; leurs mélanges.

Ces huiles peuvent être présentes en une teneur allant de 0,01 à 90%, et mieux de 0,1 à 85% en poids, par rapport au poids total de la composition.

La composition selon l'invention peut également comprendre un ou plusieurs solvants organiques, physiologiquement acceptables.

Ces solvants peuvent être généralement présents en une teneur allant de 0,1 à 90%, de préférence de 0,5 à 85%, de préférence encore de 10 à 80% en poids, par rapport au poids total de la composition, et mieux de 30 à 50 %.

On peut notamment citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone; les éthers de propylène glycol liquides à température ambiante tels que le monométhyléther de propylène glycol, l'acétate de monométhyl éther de propylène glycol, le mono n-butyl éther de dipropylène glycol; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à 25 °C tels que le diéthyléther, le diméthyléther ou le dichlorodiéthyléther; les alcanes liquides à 25°C tels que le décane, l'heptane, le dodécane, l'isododécane, le cyclohexane; les composés cycliques aromatiques liquides à 25°C tels que le toluène et le xylène; les aldéhydes liquides à 25°C tels que le benzaldéhyde, l'acétaldéhyde et leurs mélanges.

Par cire au sens de la présente invention, on entend un composé lipophile, solide à température ambiante (25°C), à changement d'état solide/liquide réversible, ayant un point de fusion supérieur ou égal à 25°C pouvant aller jusqu'à 120°C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent un point de fusion supérieur à 30°C et mieux supérieur à 45°C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène

ou de Fischer Tropsch, les cires de silicones comme les alkyl ou alkoxydiméthicone ayant de 16 à 45 atomes de carbone. Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

Par corps gras pâteux, on entend un produit visqueux contenant une fraction liquide et une fraction solide. On entend notamment des corps gras ayant un point de fusion allant de 20 à 55°C, de préférence 25 à 45°C, et/ou une viscosité à 40°C allant de 0,1 à 40 Pa.s (1 à 400 poises), de préférence 0,5 à 25 Pa.s, mesurée au Contraves TV ou Rhéomat 80. L'homme du métier peut choisir le mobile permettant de mesurer la viscosité, parmi les mobiles MS-r3 et MS-r4, sur la base de ses connaissances générales, de manière à pouvoir réaliser la mesure de la viscosité du composé pâteux testé.

Les valeurs de point de fusion correspondent, selon l'invention, au pic de fusion mesurée par la méthode "Differential Scanning Calorimetry" avec une montée en température de 5 ou 10 °C/min.

De préférence, ces corps gras sont des composés hydrocarbonés (contenant principalement des atomes de carbone et d'hydrogène et éventuellement des groupements ester), éventuellement de type polymérique; ils peuvent également être choisis parmi les composés siliconés et/ou fluorés; ils peuvent aussi se présenter sous forme d'un mélange de composés hydrocarbonés et/ou siliconés et/ou fluorés. Dans le cas d'un mélange de différents corps gras pâteux, on utilise de préférence les composés pâteux hydrocarbonés en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle; des esters d'acides ou d'alcools gras, notamment ceux ayant 20 à 65 atomes de carbone comme le citrate de tri-isostéaryle ou de cétyle; le propionate d'arachidyle; le polylaurate de vinyle; les esters du cholestérol comme les triglycérides d'origine végétale tels que les huiles végétales hydrogénées, les polyesters visqueux comme l'acide poly(12-hydroxystéarique) et leurs mélanges. Comme triglycérides d'origine végétale, on peut utiliser les dérivés d'huile de ricin hydrogénée.

On peut aussi citer les corps gras pâteux siliconés tels que les polydiméthylsiloxanes (PDMS) ayant des chaînes pendantes du type alkyle ou alcoxy ayant de 8 à 24 atomes de carbone, comme les stéaryl diméthicones.

La nature et la quantité des corps solides sont fonction des propriétés mécaniques et des textures recherchées. A titre indicatif, la composition peut contenir de 0,1 à 50% en poids de cires, par rapport au poids total de la composition et mieux de 1 à 30% en poids.

La composition selon l'invention peut en outre comprendre, dans une phase particulaire, des pigments et/ou des nacres et/ou des charges habituellement utilisés dans les compositions cosmétiques.

La composition peut également comprendre d'autres matières colorantes choisies parmi les colorants hydrosolubles et/ou les colorants liposolubles bien connus de l'homme du métier.

Par pigments, il faut comprendre des particules de toute forme, blanches ou colorées, minérales ou organiques, insolubles dans le milieu physiologique, destinées à colorer la composition.

Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Par nacres, il faut comprendre des particules de toute forme irisées, notamment produites par certains mollusques dans leur coquille ou bien synthétisées.

Les pigments peuvent être présents dans la composition à raison de 0,01 à 25% en poids de la composition finale, et de préférence à raison de 3 à 10% en poids. Ils peuvent être blancs ou colorés, minéraux ou organiques,. On peut citer les oxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium. On peut encore citer les pigments D&C et les laques couramment employées pour conférer aux lèvres et à la peau un effet de maquillage, qui sont des sels de calcium, de baryum, d'aluminium, de strontium ou de zirconium.

Les nacres peuvent être présentes dans la composition à raison de 0,01 à 20% en poids, de préférence à un taux de l'ordre de 3 à 10% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Parmi les colorants, liposolubles ou hydrosolubles, qui peuvent être présents dans la composition, seul ou en mélange, à raison de 0,001 à 15% en poids, de préférence 0,01 à 5% en poids et notamment de 0,1 à 2% en poids, par rapport au poids total de la composition, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène, le carmin de cochenille, les colorants halogéno-acides, azoïques, anthraquinoniques, le sulfate de cuivre ou de fer, le brun Soudan, le rouge Soudan et le rocou, ainsi que le jus de betterave et le carotène.

La composition selon l'invention peut comprendre en outre en outre une ou plusieurs charges, notamment en une teneur

allant de 0,01% à 50% en poids, par rapport au poids total de la composition, de préférence allant de 0,02% à 30% en poids. Les charges peuvent être minérales ou organiques de toute forme, plaquettaires, sphériques ou oblongues. On peut citer le talc, le mica, la silice, le kaolin, les poudres de polyamide (Nylon®), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition selon l'invention peut également comprendre des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, les agents auxiliaires de filmification, ou leurs mélanges.

Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; une poudre libre, compacte ou coulée; une pâte anhydre. Cette composition peut avoir l'aspect d'une lotion, d'une crème, d'une pommade, d'une pâte souple, d'un onguent, d'une mousse, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin et/ou de maquillage de la peau du corps ou du visage, des lèvres, des ongles, des cils, des sourcils et/ou des cheveux, d'un produit solaire ou autobronzant, d'un produit capillaire pour le soin, le traitement, la mise en forme, le maquillage ou la coloration des cheveux.

Elle peut ainsi se présenter sous la forme d'une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux).

Elle peut également se présenter sous forme d'une composition de protection ou de soin de la peau du visage, du cou, des mains ou du corps, notamment une composition anti-rides, une composition hydratante ou traitante; une composition anti-solaire ou de bronzage artificiel.

Elle peut encore se présenter sous forme d'un produit capillaire, notamment pour la coloration, le maintien de la coiffure, la mise en forme des cheveux, le soin, le traitement ou le nettoyage des cheveux, telle que des shampooings, des gels, des lotions de mise en plis, des lotions pour le brushing, des compositions de fixation et de coiffage telles que les laques ou spray.

De préférence, la composition cosmétique selon l'invention se présente sous la forme d'un produit de maquillage, notamment d'un fond de teint, ou d'un produit de soin tel qu'une crème de soin pour le visage ou un produit solaire.

L'invention a également pour objet un procédé de traitement cosmétique, notamment de maquillage ou de soin des matières kératiniques, telles que la peau du corps ou du visage, des lèvres, des ongles, des cils, des sourcils et/ou des cheveux, comprenant l'application sur lesdites matières d'une composition cosmétique telle que définie précédemment.

L'invention est illustrée plus en détail dans les exemples suivants.

Brillance mesurée au brillancemètre sur un dépôt sec de polymère

La brillance peut être mesurée à l'aide d'un brillancemètre, de manière conventionnelle par la méthode suivante.
Sur une carte de contraste de marque LENETA et de référence FORM 1A PENOPAC, on étale une couche de 50 µm d'épaisseur d'une solution du polymère à 50% dans l'isododécane à l'aide d'un étaleur automatique. La couche recouvre au moins le fond noir de la carte. On laisse sécher le dépôt 24 heures à une température de 25°C, puis on procède à la mesure de la brillance à 20° sur le fond noir à l'aide d'un brillancemètre de marque DR LANGE, REF03.
On procède également à la mesure de la brillance à 60°, puis à 85°, comme précédemment.
Une mesure à 20° inférieure à environ 50 donne une matité jugée acceptable, et très satisfaisante si la mesure est inférieure à 40.

Tenue à l'huile des polymères par mesure de l'aspect collant

Elle est déterminée avec une goutte d'huile d'olive mise sur un film de polymère sec.
Un film de polymère est réalisé à partir d'une solution de polymère à 20% dans l'isododécane; 0,5 ml est étalé sur une plaque de verre de 2,5 x 7,5 cm et laissé sécher à température ambiante (25°C) pendant 24 heures. Ensuite, 1 ml d'huile d'olive est étalé sur le film de polymère.
Après 1 heure, l'excès d'huile est essuyé du film et l'aspect collant est évalué au toucher, par comparaison avec le polymère comparatif.
Une note de '***' ou '3*' est donnée lorsque l'on décolle la plaque avec le doigt (très collant).
Une note de '0' signifie : pas de collant détecté.
Le collant traduit la sensibilité du polymère à l'huile d'olive. Plus le polymère est collant en présence d'huile, plus il est sensible à l'huile et donc plus le dépôt sera facilement altéré, par exemple lors des repas (en présence d'huile alimentaire) ou par le sébum. Il en résulte une moins bonne tenue du polymère sur la peau. Il en résulte aussi une diminution du confort : plus le film est collant, plus la composition est inconfortable à porter.

Contraintes internes

Un film de polymère est réalisé à partir d'une solution de polymère à 20% dans l'isododécane; on étale 30 microlitres sur une bande de nitrile de 6 x 1 cm et on laisse sécher à température ambiante (25°C) pendant 3 heures.
On observe visuellement la déformation du support après séchage.
Une note de 0 signifie que le support ne s'est pas déformé lors du séchage.
Une note de +++ signifie que le support est presque plié (complètement déformé) après séchage.
Les contraintes internes (CI) reflètent le confort du point de vue mécanique: lorsqu'il n'y a pas de déformation du support lors du séchage, on peut penser qu'il n'y aura pas d'inconfort lors du séchage de la composition après son application sur la peau.

**Exemple 1**

On introduit 30 g d'isododécane dans un tricol de 250 ml, puis on chauffe jusqu'à 90°C. On ajoute en 30 minutes, 4,5 g d'acrylate d'isobornyle, 4,5 g de méthacrylate d'isobornyle et 0,37 g d'amorceur 2,5-bis(2-éthylhexanoylperoxy)-2,5-diméthylhexane (Trigonox® 141 d'Akzo Nobel). On maintient le tout à 90°C pendant 1 h 30.
On introduit ensuite au mélange précédent, à 90°C et en 30 minutes, 21 g de MPTS et 0,29 g de 2,5-bis(2-éthylhexanoylperoxy)-2,5-diméthylhexane. Le mélange est maintenu 3 heures à 90°C, puis l'ensemble est refroidi à 25°C.
On obtient une solution à 51,5% de matière sèche de polymère dans l'isododécane. Ledit polymère comprend une 1ère séquence comprenant les (méth)acrylates d'isobornyle et une deuxième séquence comprenant le monomère siliconé.

**Exemple 2**

On introduit 30 g d'isododécane dans un tricol de 250 ml, puis on chauffe jusqu'à 90°C. On ajoute en 30 minutes, 7,5 g d'acrylate d'isobornyle, 7,5 g de méthacrylate d'isobornyle et 0,30 g d'amorceur 2,5-bis(2-éthylhexanoylperoxy)-2,5-diméthylhexane (Trigonox® 141 d'Akzo Nobel). On maintient le tout à 90°C pendant 1 h 30.
On introduit ensuite au mélange précédent, à 90°C et en 30 minutes, 15,2 g de MPTS et 0,20 g de 2,5-bis(2-éthylhexanoylperoxy)-2,5-diméthylhexane. Le mélange est maintenu 3 heures à 90°C, puis l'ensemble est refroidi à 25°C.
On obtient une solution à 51,2% de matière sèche de polymère dans l'isododécane. Ledit polymère comprend une 1ère séquence comprenant les (méth)acrylates d'isobornyle et une deuxième séquence comprenant le monomère siliconé.

**Exemple 3**

On introduit 60 g d'isododécane dans un tricol de 250 ml, puis on chauffe jusqu'à 90°C. On ajoute en 30 minutes, 21 g d'acrylate d'isobornyle, 21 g de méthacrylate d'isobornyle et 0,36 g d'amorceur 2,5-bis(2-éthylhexanoylperoxy)-2,5-diméthylhexane (Trigonox® 141 d'Akzo Nobel). On maintient le tout à 90°C pendant 2 h 30.
On introduit ensuite au mélange précédent, à 90°C et en 30 minutes, 18 g de MPTS et 0,25 g de 2,5-bis(2-éthylhexanoylperoxy)-2,5-diméthylhexane. Le mélange est maintenu 3 heures à 90°C, puis l'ensemble est refroidi à 25°C.
On obtient une solution à 53,1% de matière sèche de polymère dans l'isododécane. Ledit polymère comprend une 1ère séquence comprenant les (méth)acrylates d'isobornyle et une deuxième séquence comprenant le monomère siliconé.

**Exemple 4**

On introduit 30 g d'isododécane dans un tricol de 250 ml, puis on chauffe jusqu'à 90°C. On ajoute en 30 minutes, 21 g de méthacrylate d'isobornyle et 0,19g d'amorceur 2,5-bis(2-éthylhexanoylperoxy)-2,5-diméthylhexane (Trigonox® 141 d'Akzo Nobel). On maintient le tout à 90°C pendant 1 h 30.
On introduit ensuite au mélange précédent, à 90°C et en 30 minutes, 9 g de MPTS et 0,12 g de 2,5-bis(2-éthylhexanoylperoxy)-2,5-diméthylhexane. Le mélange est maintenu 3 heures à 90°C, puis l'ensemble est refroidi à 25°C.
On obtient une solution à 52,8% de matière sèche de polymère dans l'isododécane. Ledit polymère comprend une 1ère séquence comprenant le méthacrylate d'isobornyle et une deuxième séquence comprenant le monomère siliconé.

**Exemple 5**

On introduit 30 g d'isododécane dans un tricol de 250 ml, puis on chauffe jusqu'à 90°C. On ajoute en 30 minutes, 15 g de méthacrylate d'isobornyle et 0,30g d'amorceur 2,5-bis(2-éthylhexanoylperoxy)-2,5-diméthylhexane (Trigonox® 141 d'Akzo Nobel). On maintient le tout à 90°C pendant 1 h 30.
On introduit ensuite au mélange précédent, à 90°C et en 30 minutes, 15 g de MPTS et 0,20 g de 2,5-bis(2-éthylhexanoylperoxy)-2,5-diméthylhexane. Le mélange est maintenu 3 heures à 90°C, puis l'ensemble est refroidi à 25°C.
On obtient une solution à 51,7% de matière sèche de polymère dans l'isododécane. Ledit polymère comprend une 1ère séquence comprenant le méthacrylate d'isobornyle et une deuxième séquence comprenant le monomère siliconé.

**Exemple 6**

On introduit 30 g d'isododécane dans un tricol de 250 ml, puis on chauffe jusqu'à 90°C. On ajoute en 30 minutes, 7,5 g d'acrylate d'isobornyle, 7,5 g de méthacrylate d'isobornyle et 0,33 g d'amorceur peroxy-2-ethylhexanoate de tert-butyle (Trigonox® 21S d'Akzo Nobel). On maintient le tout à 90°C pendant 1 h 30.

On introduit ensuite au mélange précédent, à 90°C et en 30 minutes, 13,5 g de MPTS, 1,5 g d'acide acrylique et 0,22 g de Trigonox 21 S. Le mélange est maintenu 3 heures à 90°C, puis l'ensemble est refroidi à 25°C.
On obtient une solution à 56,2% de matière sèche de polymère dans l'isododécane. Ledit polymère comprend une 1ère séquence comprenant les (méth)acrylates d'isobornyle et une deuxième séquence comprenant le monomère siliconé et l'acide acrylique.

**Exemple 7**

On introduit 30 g d'isododécane dans un tricol de 250 ml, puis on chauffe jusqu'à 90°C. On ajoute en 30 minutes, 4,5 g d'acrylate d'isobornyle, 4,5 g de méthacrylate d'isobornyle et 0,37 g d'amorceur 2,5-bis(2-éthylhexanoylperoxy)-2,5-diméthylhexane (Trigonox® 141 d'Akzo Nobel). On maintient le tout à 90°C pendant 1 h 30.
On introduit ensuite au mélange précédent, à 90°C et en 30 minutes, 19,5 g de MPTS, 1,5 g d'acide acrylique et 0,29 g de 2,5-bis(2-éthylhexanoylperoxy)-2,5-diméthylhexane. Le mélange est maintenu 3 heures à 90°C, puis l'ensemble est refroidi à 25 °C.
On obtient une solution à 55,4% de matière sèche de polymère dans l'isododécane. Ledit polymère comprend une 1ère séquence comprenant les (méth)acrylates d'isobornyle et une deuxième séquence comprenant le monomère siliconé et l'acide acrylique.

**Exemple 8**

On introduit 30 g d'isododécane dans un tricol de 250 ml, puis on chauffe jusqu'à 90°C. On ajoute en 30 minutes, 9 g de méthacrylate d'isobornyle et 0,37 g d'amorceur 2,5-bis(2-éthylhexanoylperoxy)-2,5-diméthylhexane (Trigonox® 141 d'Akzo Nobel). On maintient le tout à 90°C pendant 1 h 30.

On introduit ensuite au mélange précédent, à 90°C et en 30 minutes, 21 g de MPTS et 0,28 g de 2,5-bis(2-éthylhexanoylperoxy)-2,5-diméthylhexane. Le mélange est maintenu 3 heures à 90°C, puis l'ensemble est refroidi à 25°C.
On obtient une solution à 52,3% de matière sèche de polymère dans l'isododécane. Ledit polymère comprend une 1ère séquence comprenant le méthacrylate d'isobornyle et une deuxième séquence comprenant le monomère siliconé.

**Exemple 9**

On introduit 30 g d'isododécane dans un tricol de 250 ml, puis on chauffe jusqu'à 90°C. On ajoute en 1 heure, 10,5 g d'acrylate d'isobornyle, 10,5 g de méthacrylate d'isobornyle et 0,19 g d'amorceur peroxy-2-ethylhexanoate de tert-butyle (Trigonox® 21S d'Akzo Nobel). On maintient le tout à 90°C pendant 1 h 30.
On introduit ensuite au mélange précédent, à 90°C et en 30 minutes, 8,25 g de MPTS, 0,75 g d'acide acrylique et 0,13 g de Trigonox 21 S. Le mélange est maintenu 3 heures à 90°C, puis l'ensemble est refroidi à 25°C.
On obtient une solution à 54,6% de matière sèche de polymère dans l'isododécane. Ledit polymère comprend une 1ère séquence comprenant les (méth)acrylates d'isobornyle et une deuxième séquence comprenant le monomère siliconé et l'acide acrylique.

**Exemple comparatif (hors invention)**

On introduit 100 g d'isododécane dans un réacteur de 1 litre, puis on augmente la température de façon à passer de la température ambiante (25°C) à 90 °C en une heure.
On ajoute ensuite, à 90°C et en 1 heure, 105 g d'acrylate d'isobornyle, 105 g de méthacrylate d'isobornyle, 110 g d'isododécane et 1,8 g de 2.5-bis(2-éthylhexanoylperoxy)-2.5-diméthylhexane (Trigonox® 141 d'Akzo Nobel). Le mélange est maintenu 1 h 30 à 90°C.
On introduit ensuite au mélange précédent, toujours à 90°C et en 30 minutes, 90 g d'acrylate d'isobutyle, 90 g d'isododécane et 1,2 g de 2.5-bis(2-éthylhexanoylperoxy)-2.5-diméthylhexane. Le mélange est maintenu 3 heures à 90°C, puis l'ensemble est refroidi.
On obtient une solution à 50% de matière active en polymère dans l'isododécane. Ledit polymère comprend une 1ère séquence comprenant les (méth)acrylates d'isobornyle et une deuxième séquence comprenant l'acrylate d'isobutyle.

**Exemple 10**

On caractérise les films obtenus à partir des polymères de l'invention des exemples 1 à 9, ainsi qu'avec le polymère comparatif préparé ci-dessus.
Film : on observe visuellement le collant, la brillance et le caractère cassant d'un film seul de polymère.
On détermine, selon les méthodes indiquées plus haut, la brillance, le collant (tenue à l'huile d'olive) et les contraintes internes du polymère.
On obtient les résultats suivants :

| | Exemple 1 | Exemple 2 | Exemple 3 | Exemple 4 | Exemple 5 |
|---|---|---|---|---|---|
| % A. Isob. | 15 | 25 | 35 | -- | -- |
| % M. Isob. | 15 | 25 | 35 | 70 | 50 |
| % MPTS | 70 | 50 | 30 | 30 | 50 |
| Mw (g/mol) | 67 700 | 61 900 | 67 800 | 95400 | 68 400 |
| Ip | 7,85 | 5,99 | 4,49 | 3,83 | 2,82 |
| ES (%) | 51,5% | 51,2% | 53,1% | 52,8% | 51,7% |
| Film seul (observation visuelle) | un peu brillant | mat | Blanc | mat | Mat |
| | un peu collant | non collant | non collant | non collant | non collant |
| | non cassant | non cassant | très légèrement cassant | légèrement cassant | très légèrement cassant |
| Brillance | 20° :46 | 20° :1 | 20° :1,6 | 20° :1,2 | 20° :1,2 |
| | 60° :76 | 60° :4,4 | 60° :7,5 | 60° :2,7 | 60° :3,3 |
| | 85° :93,4 | 85° :13,2 | 85° :23,8 | 85° :3,7 | 85° :6,6 |

(suite)

| | Exemple 1 | Exemple 2 | Exemple 3 | Exemple 4 | Exemple 5 |
|---|---|---|---|---|---|
| Collant | ++ | + | + | 0 | + |
| Contraintes internes | 0 | 0 | ++ | + | 0 |

| | Exemple 6 | Exemple 7 | Exemple 8 | Exemple 9 | Comparatif |
|---|---|---|---|---|---|
| % A. Isob. | 25 | 15 | -- | 35 | 35 |
| % M. Isob. | 25 | 15 | 30 | 35 | 35 |
| % MPTS | 45 | 65 | 70 | 27,5 | -- |
| % AA | 5 | 5 | -- | 2,5 | -- |
| % A. Ibut. | -- | -- | -- | -- | 30 |
| Mw (g/mol) | 168 600 | 663 600 | 86 500 | 104 000 | 100 300 |
| Ip | 8,22 | 32,75 | 3,69 | 4,52 | 4,40 |
| ES (%) | 56,2% | 55,4% | 52,3% | 54,6% | 50% |
| Film seul (observation visuelle) | mat, un peu opaque | opaque, légèrement brillant | mat | mat | brillant |
| | non collant | non collant | peu collant | Non collant | non collant |
| | non cassant | très légèrement cassant | non cassant | Non cassant | cassant |
| Brillance | 20° : 2,4 | 20° : 44,1 | 20° : 8,5 | 20° : 5,2 | 20° : 71,3 |
| | 60° :15,2 | 60° : 75,1 | 60° : 37 | 60° : 26,2 | 60° : 86,6 |
| | 85° :23,2 | 85° : 95,5 | 85° : 62,9 | 85° : 35,9 | |
| Collant | 0 | + | ++ | 0 | +++ |
| Contraintes internes | + | 0 | 0 | + | +++ |
| A. Isob. : acrylate d'isobornyle<br>M. Isob. : méthacrylate d'isobornyle<br>MPTS : méthacryloxypropyltris(trimethylsiloxy)silane<br>AA : acide acrylique<br>A. Ibut : acrylate d'isobutyle<br>ES : extrait sec | | | | | |

**Exemple 11**

On prépare un fond de teint de la manière suivante :

- les pigments (phase A3) sont broyés 3 fois à la broyeuse tricylindre dans le cyclopentasiloxane;
- on mélange les constituants de la phase A1 sous agitation Moritz à température ambiante puis on ajoute les phases A2, A3, A4 et A5 en prenant soin de bien homogénéiser entre chaque phase et d'augmenter la vitesse d'agitation si nécessaire;
- on ajoute ensuite progressivement la phase B en augmentant la vitesse d'agitation si nécessaire, et on laisse sous agitation pendant 10 minutes.

| Phase | Taux (%) | Composé |
|---|---|---|
| A1 | 1,8 | Bis PEG/PPG-14/14 diméthicone |
| | 0,6 | Isostearyl diglyceryl succinate |
| | Qsp 100% | Isododécane |
| | 5,0 | Cyclopentasiloxane (D5) |
| A2 | 4,25 | Polymère de l'ex.7 (soit 2,2% MS de polymère) |
| A3 | 5,0 | Cyclopentasiloxane (D5) |
| | 3,5 | Oxydes de fer enrobés |
| | 6,8 | Dioxyde de titane enrobé |
| A4 | 3,0 | Gomme de silicone dans la D5 |
| A5 | 8,0 | Nylon 12 |
| B | 40,0 | Eau |
| | Qs | Conservateur |
| | 0,7 | Sulfate de magnésium |

Cette composition de fond de teint, appliqué sur le visage, présente des propriétés de confort et de matité satisfaisantes.

**Revendications**

**1.** Composition cosmétique ou pharmaceutique comprenant, dans un milieu physiologiquement acceptable, au moins un polymère séquencé comprenant au moins une première séquence et au moins une deuxième séquence, dans lequel au moins l'une des séquences comprend de 35 à 100% en poids, par rapport au poids de ladite séquence, d'au moins un monomère siliconé choisi parmi, seul ou en mélange, les monomères suivants :

- (i) les monomères éthyléniques dont le groupement ester contient des silanes et/ou des siloxanes, de formule :

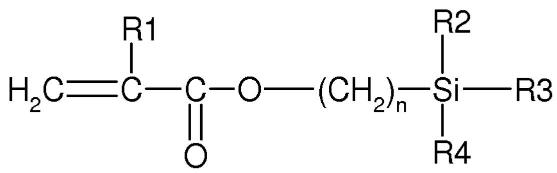

dans laquelle :

- R1= H ou méthyle,
- R2, R3, R4, identiques ou différents, représentent des groupes alkyles en C1 à C6 ou le groupe -OSi $(R5)_3$ avec R5= méthyle ou éthyle;
- n est un nombre entier de 1 à 10;

- (ii) les macromonomères PDMS, ou polydiméthylsiloxanes à groupement terminal monoacryloyloxy ou monométhacryloyloxy, de formule suivante

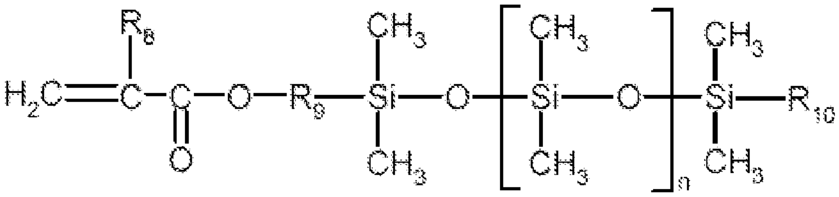

dans laquelle :

- R8 désigne un atome d'hydrogène ou un groupement méthyle;
- R9 désigne un groupe hydrocarboné divalent, linéaire ou ramifié, ayant de 1 à 10 atomes de carbone et contenant éventuellement une ou deux liaisons éther - O- ;
- R10 désigne un groupe alkyle linéaire ou ramifié, ayant de 1 à 10 atomes de carbone;
- n désigne un nombre entier allant de 1 à 300.

- (iii) les monomères éthyléniques dont le groupement ester contient des carboxysilanes dendrimères de formule :

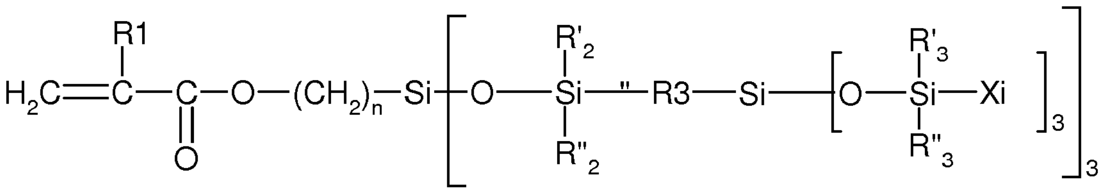

dans laquelle :

- R1= H ou méthyle;
- n est un nombre entier de 1 à 10;
- R'2, R"2, R'3 et R"3, identiques ou différents, représentent un groupe alkyle en C1 à C10;
- R3 est un groupe divalent alkylène en C2 à C10;
- i est un entier compris entre 1 et 10; et

  - quand i = 2 à 10, X(i), identique ou différent, représente un groupe $-R4-Si-[O-(R'3)(R''3)-X(i-1)]_3$, avec R4, identique ou différent, représentant un groupe divalent alkylène en C2 à C10;
  - et X(1) représente H ou un groupe alkyle en C1-C10;

- (iv) les monomères éthyléniques de type POSS ou POS de structure :

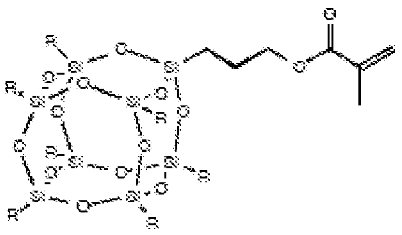

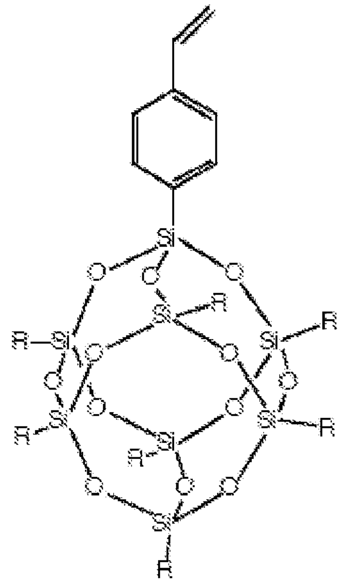

dans lesquelles R, identique ou différent, est un groupe alkyle linéaire en C1 à C10 ou cyclique en C3 à C12.

**2.** Composition selon la revendication 1, dans laquelle les monomères siliconés sont choisis parmi, seuls ou en mélange, les monomères suivants :

- le (méth)acryloxypropyltris(trimethylsiloxy)silane, le (méth)acryloxypropyl-bis(trimethylsiloxy)méthylsilane,
- le (méth)acryloxyméthyltris(trimethylsiloxy)silane, le (méth)acryloxyméthyl-bis(trimethylsiloxy)méthylsilane;
- le (méth)acryloxypropryltrimethoxysilane;
- les monomères suivants, dans lesquels R1 = H ou méthyle :

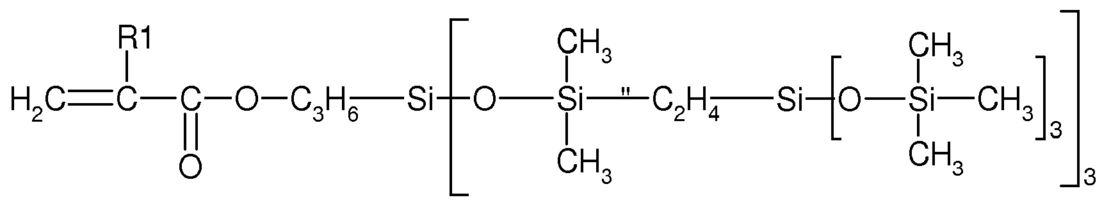

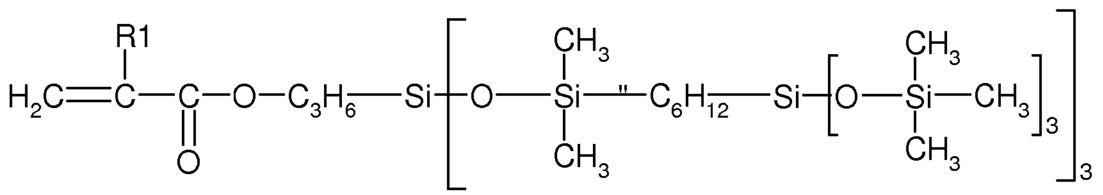

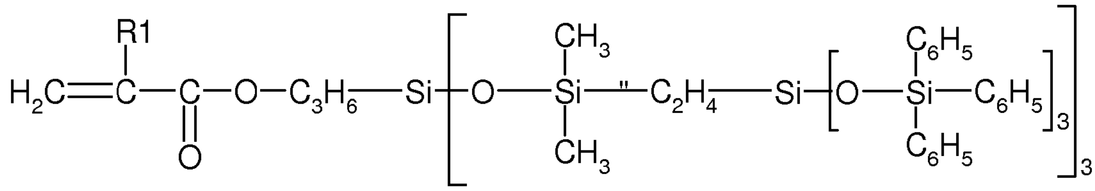

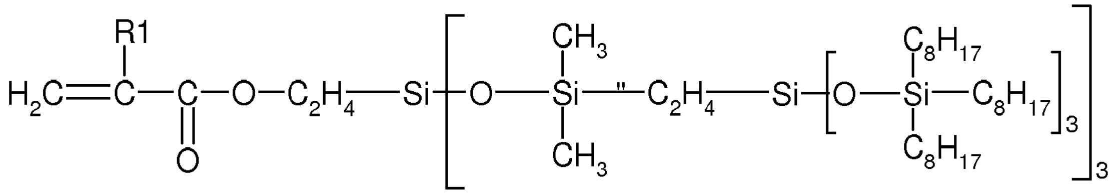

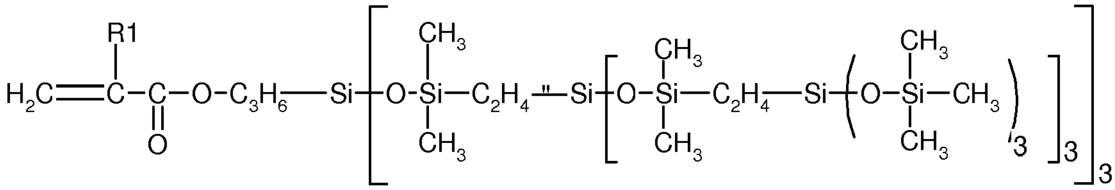

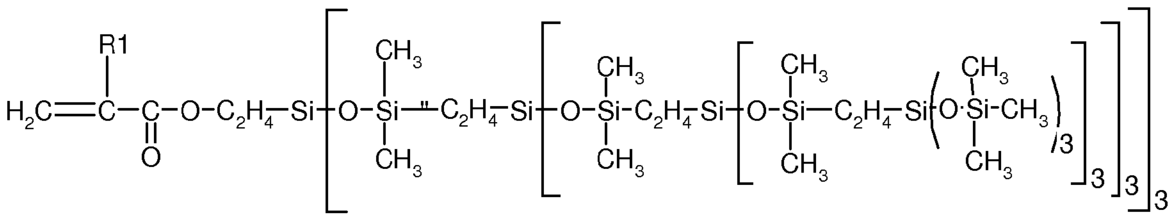

3. Composition selon l'une des revendications précédentes, dans laquelle les monomères siliconés représentent 50% à 99% en poids, voire 60% à 95% en poids, préférentiellement 70% à 90% en poids, du poids total de la séquence les comprenant.

4. Composition selon l'une des revendications précédentes, dans laquelle les monomères siliconés représentent 20 à 90% en poids, notamment 25 à 80% en poids, encore mieux 30 à 75% en poids, voire 35 à 70% en poids, du poids total du polymère.

5. Composition selon l'une des revendications précédentes, dans laquelle le polymère comprend en outre 10 à 80% en poids, notamment 20 à 75% en poids, encore mieux 25 à 70% en poids, voire 30 à 65% en poids, d'un ou plusieurs monomères carbonés choisis.

6. Composition selon la revendication 5, dans laquelle le/les monomères carbonés choisis sont choisis parmi, seuls ou en mélange, les monomères suivants :

- les méthacrylates de formule $CH_2 = C(CH_3)\text{-}COOR1$ dans laquelle R1 représente un groupe alkyle linéaire ou ramifié en C8-C22; ou bien un groupe alkyle cyclique ayant 8 à 30 atomes de carbone; ou encore R1 représente le groupe tertiobutyle;
- les acrylates de formule $CH_2 = CH\text{-}COOR2$ dans laquelle R2 représente un groupe alkyle linéaire ou ramifié

en C8-C22; ou bien un groupe alkyle cyclique ayant 8 à 30 atomes de carbone; ou encore R2 représente un groupe isobutyle;
- les (méth)acrylamides de formule $CH_2=C(CH_3)-CONR3R4$ ou $CH_2=CH-CONR3R4$, dans lesquelles R3 représente un atome d'hydrogène ou un groupe alkyle linéaire ou ramifié en C1-C12, et R4 représente un groupe alkyle en C8 à C12 linéaire ou ramifié;
- les di-n-alkylitaconates de formule $CH_2=C(CH_2-COO(CH_2)_n-CH_3)COO(CH_2)_n-CH_3$, avec n étant un entier supérieur ou égal à 5;
- les esters vinyliques de formule $CH_2=CHOCOR5$ où R5 représente un groupe alkyle en C8 à C22 linéaire ou ramifié;
- les éthers de vinyle de formule $CH_2=CH-OR6$ dans laquelle R6 représente un groupe alkyle linéaire ou ramifié, comprenant de 8 à 22 atomes de carbone;
- les homopolymères et les copolymères (méth)acrylate d'alkyle linéaire ou ramifié en C6-C22, présentant un groupe terminal polymérisable choisi parmi les groupes vinyle ou (méth)acrylate;
- les polyoléfines ayant un groupe terminal à insaturation éthylénique, en particulier un groupement terminal (méth)acrylate.

**7.** Composition selon l'une des revendications 5 à 6, dans laquelle les monomères carbonés choisis sont choisis parmi, seuls ou en mélange, les méthacrylates de lauryle, de béhényle, de stéaryle, d'isobornyle, de tertiobutyle; les acrylates de lauryle, de béhényle, de 2-éthylhexyle, de stéaryle, d'isobornyle, d'isobutyle; les macromonomères à base de poly(acrylate d'éthyl-2-hexyle) ou de poly(acrylate de dodécyle) à extrémité mono(méth)acrylate; des macromonomères éthylène/butylène et éthylène/propylène à groupement terminal réactif (méth)acrylate.

**8.** Composition selon l'une des revendications précédentes, dans laquelle le polymère comprend en outre au moins un monomère additionnel choisi parmi, seul ou en mélange, les monomères suivants :

- (i) les hydrocarbures éthyléniques ayant 2 à 10 carbones;
- (ii) les (méth)acrylates de formule $CH_2 = CHCOOR'_3$ ou $CH_2 = C(CH_3)COOR'_3$
dans lesquelles $R'_3$ représente :

  - un groupe alkyle linéaire ou ramifié, de 1 à 7 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle ou les atomes d'halogène (Cl, Br, I et F);
  - un groupe cycloalkyle en $C_3$ à $C_7$,
  - un groupe aryle en $C_3$ à $C_{20}$,
  - un groupe aralkyle en $C_4$ à $C_{30}$ (groupe alkyle en $C_1$ à $C_8$),
  - un groupe hétérocyclique de 4 à 12 chaînons contenant un ou plusieurs hétéroatomes choisis parmi O, N, et S, le cycle étant aromatique ou non,
  - un groupe hétérocycloalkyle (alkyle en C1-C4),
  lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-4, linéaires ou ramifiés dans lesquels se trouve(nt) éventuellement intercalé (s) un ou plusieurs hétéroatomes choisis parmi O, N, S et P, lesdits groupes alkyle pouvant, en outre, être éventuellement substitués par un ou plusieurs substituants choisis parmi les groupes hydroxyle ou les atomes d'halogène,
  - $R'_3$ peut également être un groupe $-(C_2H_4O)_m-R''$, avec m = 5 à 150 et R" = H ou alkyle de $C_1$ à $C_{30}$;

ou encore le méthacrylate d'isobutyle ou l'acrylate de tertiobutyle;
- (iii) les (méth)acrylamides de formule :

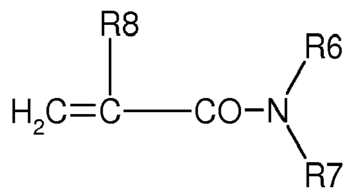

dans laquelle $R_8$ désigne H ou méthyle; et $R_7$ et $R_6$ identiques ou différents représentent :

- un atome d'hydrogène,
- un groupe alkyle linéaire ou ramifié, de 1 à 22 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F);
- un groupe cycloalkyle en $C_3$ à $C_{12}$,
- un groupe aryle en $C_3$ à $C_{20}$,
- un groupe aralkyle en $C_4$ à $C_{30}$ (groupe alkyle en $C_1$ à $C_8$),
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4),

lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F);
- (iv) les composés vinyliques de formules :

$$CH_2=CH\text{-}R_9,\ CH_2=CH\text{-}CH_2\text{-}R_9 \text{ ou } CH_2=C(CH_3)\text{-}CH_2\text{-}R_9$$

dans lesquelles $R_9$ est un groupe hydroxyle, halogène, $NH_2$, $OR_{14}$ où $R_{14}$ représente un groupe phényle ou un groupe alkyle en $C_1$ à $C_7$; acétamide ($NHCOCH_3$); un groupe $OCOR_{15}$ où $R_{15}$ représente un groupe alkyle de 2 à 7 carbones, linéaire ou ramifié; ou un groupe choisi parmi :

- un groupe alkyle linéaire ou ramifié, de 1 à 22 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F),
- un groupe cycloalkyle en $C_3$ à $C_{12}$,
- un groupe aryle en $C_3$ à $C_{20}$,
- un groupe aralkyle en $C_4$ à $C_{30}$ (groupe alkyle en $C_1$ à $C_8$),
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4),

lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle les atomes d'halogène;
- (v) les monomères à insaturation(s) éthylénique(s) comprenant au moins une fonction acide carboxylique, phosphorique ou sulfonique, ou anhydride, comme par exemple l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,
- (vi) les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci.

**9.** Composition selon la revendication 8, dans laquelle les monomères additionnels sont choisis parmi, seul ou en mélange, l'acide (méth)acrylique et les (méth)acrylates d'alkyle en C1-C4, ou de cycloalkyle en C3-C7, et notamment le méthacrylate de méthyle, le méthacrylate d'isobutyle; l'acrylates de tertiobutyle, l'acide acrylique et l'acide méthacrylique.

**10.** Composition selon l'une des revendications précédentes, dans laquelle lesdites première et deuxième séquences

sont reliées entre elles par une séquence (ou bloc) intermédiaire comprenant au moins un monomère constitutif de ladite première séquence et au moins un monomère constitutif de ladite deuxième séquence.

**11.** Composition selon l'une des revendications précédentes, dans laquelle le polymère est présent, seul ou en mélange, en une quantité de 0,01 à 50% en poids, de préférence 0,05 à 40% en poids, notamment 0,1 à 30% en poids, voire 0,5 à 20% en poids, et encore mieux 1 à 15% en poids, préférentiellement 1,5 à 12% en poids, par rapport au poids total de la composition.

**12.** Composition selon l'une des revendications précédentes, dans laquelle le milieu physiologiquement acceptable comprend au moins un constituant choisi parmi l'eau, les solvants organiques hydrophiles, les cires, les corps gras pâteux, les gommes et leurs mélanges; les solvants organiques lipophiles; les huiles, notamment hydrocarbonées d'origine animale ou végétale, ou siliconées; les pigments, les nacres, les charges, les colorants hydrosolubles, les colorants liposolubles; les polymères notamment filmogènes; les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents antipelliculaires, les agents propulseurs, les céramides, les agents auxiliaires de filmification, ou leurs mélanges.

**13.** Composition selon l'une des revendications précédentes, se présentant sous la forme d'un produit de soin et/ou de maquillage de la peau du corps ou du visage, des lèvres, des ongles, des cils, des sourcils et/ou des cheveux, d'un produit solaire ou autobronzant, d'un produit capillaire pour le soin, le traitement, la mise en forme, le maquillage ou la coloration des cheveux.

**14.** Procédé de traitement cosmétique, notamment de maquillage ou de soin des matières kératiniques, telles que la peau du corps ou du visage, des lèvres, des ongles, des cils, des sourcils et/ou des cheveux, comprenant l'application sur lesdites matières d'une composition cosmétique telle que définie à l'une des revendications 1 à 13.

**15.** Polymère séquencé comprenant au moins une première séquence et au moins une deuxième séquence, dans lequel au moins l'une des séquences comprend de 35 à 100% en poids, par rapport au poids de ladite séquence, d'au moins un monomère siliconé choisi parmi, seul ou en mélange, les monomères suivants :

- (i) les monomères éthyléniques dont le groupement ester contient des silanes et/ou des siloxanes, de formule :

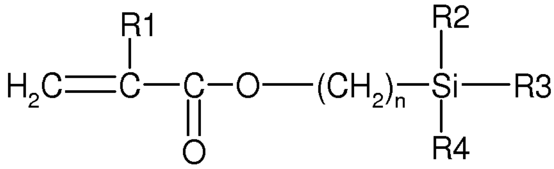

dans laquelle :

- R1= H ou méthyle,
- R2, R3, R4, identiques ou différents, représentent des groupes alkyles en C1 à C6 ou le groupe -OSi $(R5)_3$ avec R5= méthyle ou éthyle;
- n est un nombre entier de 1 à 10;

- (ii) les macromonomères PDMS, ou polydiméthylsiloxanes à groupement terminal monoacryloyloxy ou monométhacryloyloxy, de formule suivante

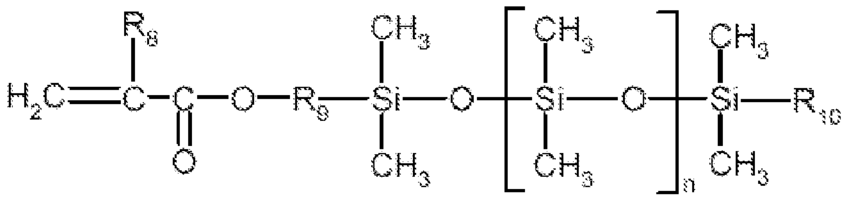

dans laquelle :

- R8 désigne un atome d'hydrogène ou un groupement méthyle;
- R9 désigne un groupe hydrocarboné divalent, linéaire ou ramifié, ayant de 1 à 10 atomes de carbone et contenant éventuellement une ou deux liaisons éther - O- ;
- R10 désigne un groupe alkyle linéaire ou ramifié, ayant de 1 à 10 atomes de carbone;
- n désigne un nombre entier allant de 1 à 300.

- (iii) les monomères éthyléniques dont le groupement ester contient des carboxysilanes dendrimères de formule :

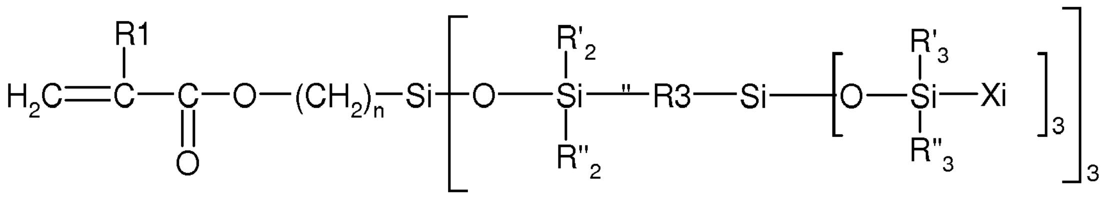

dans laquelle :

- R1= H ou méthyle;
- n est un nombre entier de 1 à 10;
- R'2, R"2, R'3 et R"3, identiques ou différents, représentent un groupe alkyle en C1 à C10;
- R3 est un groupe divalent alkylène en C2 à C10;
- i est un entier compris entre 1 et 10; et
    - quand i = 2 à 10, X(i), identique ou différent, représente un groupe -R4-Si-[O-(R'3)(R"3)-X(i-1)]$_3$, avec R4, identique ou différent, représentant un groupe divalent alkylène en C2 à C10;
    - et X(1) représente H ou un groupe alkyle en C1-C10;

- (iv) les monomères éthyléniques de type POSS ou POS de structure :

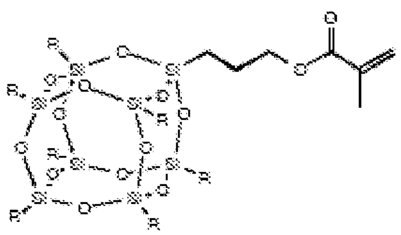

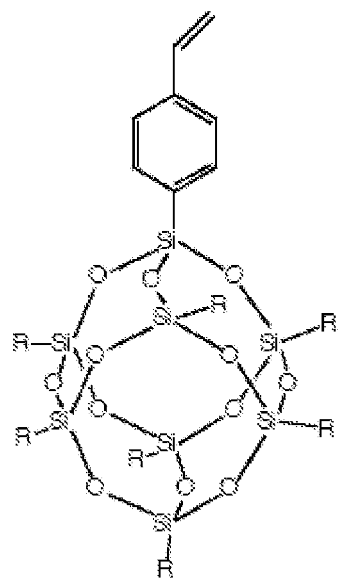

dans lesquelles R, identique ou différent, est un groupe alkyle linéaire en C1 à C10 ou cyclique en C3 à C12.

**16.** Polymère selon la revendication 15, dans lequel les monomères siliconés sont choisis parmi, seuls ou en mélange, les monomères suivants :

- le (méth)acryloxypropyltris(trimethylsiloxy)silane, le (méth)acryloxypropylbis(trimethylsiloxy)méthylsilane,
- le (méth)acryloxyméthyltris(trimethylsiloxy)silane, le (méth)acryloxyméthylbis(trimethylsiloxy)méthylsilane;
- le (méth)acryloxypropryltrimethoxysilane;
- les monomères suivants, dans lesquels R1 = H ou méthyle :

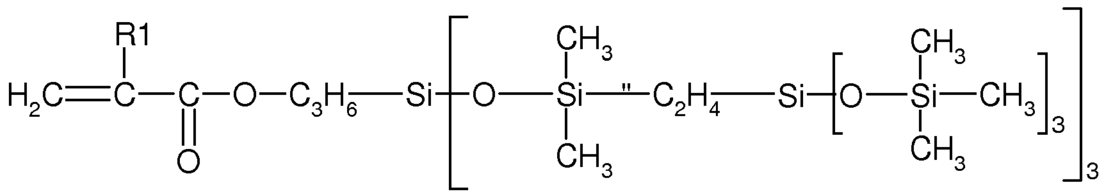

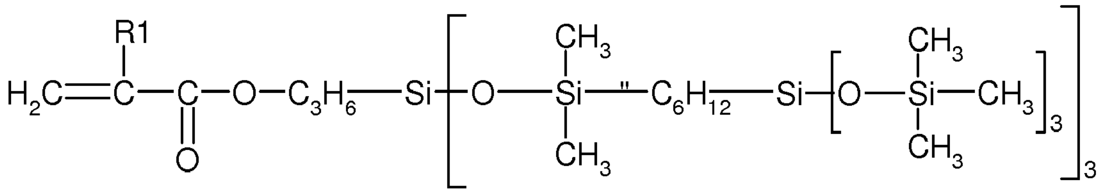

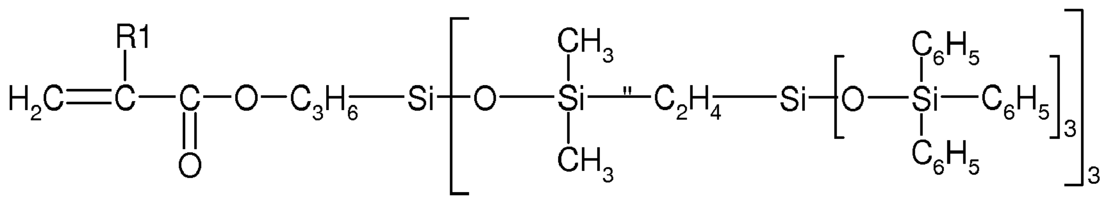

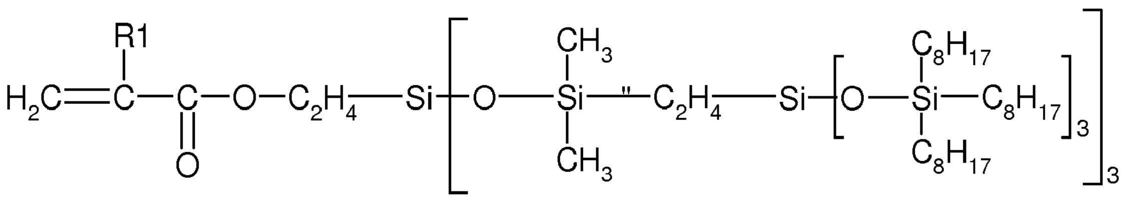

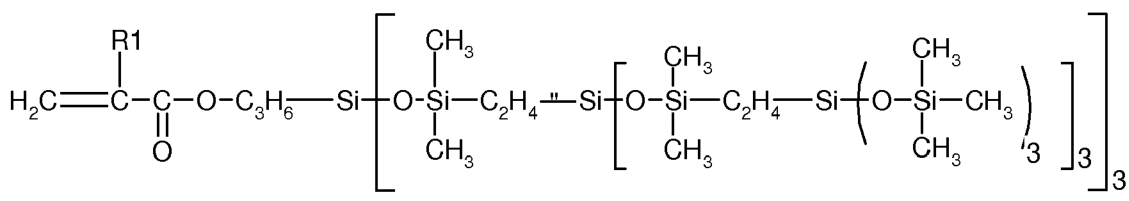

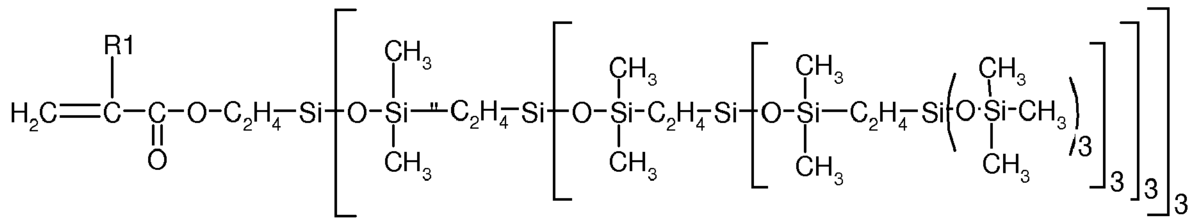

**17.** Polymère selon l'une des revendications 15 à 16, dans lequel les monomères siliconés représentent 20 à 90% en poids, notamment 25 à 80% en poids, encore mieux 30 à 75% en poids, voire 35 à 70% en poids, du poids total du polymère.

**18.** Polymère selon l'une des revendications 15 à 17, comprenant en outre 10 à 80% en poids, notamment 20 à 75% en poids, encore mieux 25 à 70% en poids, voire 30 à 65% en poids, d'un ou plusieurs monomères carbonés choisis.

**19.** Polymère selon la revendication 18, dans lequel les monomères carbonés choisis sont choisis parmi, seuls ou en mélange, les monomères suivants :

- les méthacrylates de formule $CH_2 = C(CH_3)$-COOR1 dans laquelle R1 représente un groupe alkyle linéaire ou ramifié en C8-C22; ou bien un groupe alkyle cyclique ayant 8 à 30 atomes de carbone; ou encore R1 représente le groupe tertiobutyle;
- les acrylates de formule $CH_2$ = CH-COOR2 dans laquelle R2 représente un groupe alkyle linéaire ou ramifié en C8-C22; ou bien un groupe alkyle cyclique ayant 8 à 30 atomes de carbone; ou encore R2 représente un groupe isobutyle;
- les (méth)acrylamides de formule $CH_2=C(CH_3)$-CONR3R4 ou $CH_2$=CH-CONR3R4, dans lesquelles R3 représente un atome d'hydrogène ou un groupe alkyle linéaire ou ramifié en C1-C12, et R4 représente un groupe alkyle en C8 à C12 linéaire ou ramifié;
- les di-n-alkylitaconates de formule $CH_2=C(CH_2-COO(CH_2)_n-CH_3)COO(CH_2)_n-CH_3$, avec n étant un entier supérieur ou égal à 5;
- les esters vinyliques de formule $CH_2$=CHOCOR5 où R5 représente un groupe alkyle en C8 à C22 linéaire ou ramifié;
- les éthers de vinyle de formule $CH_2$=CH-OR6 dans laquelle R6 représente un groupe alkyle linéaire ou ramifié, comprenant de 8 à 22 atomes de carbone;
- les homopolymères et les copolymères (méth)acrylate d'alkyle linéaire ou ramifié en C6-C22, présentant un groupe terminal polymérisable choisi parmi les groupes vinyle ou (méth)acrylate;
- les polyoléfines ayant un groupe terminal à insaturation éthylénique, en particulier un groupement terminal (méth)acrylate.

**20.** Polymère selon la revendication 19, dans lequel les monomères carbonés choisis sont choisis parmi, seuls ou en mélange, les méthacrylates de lauryle, de béhényle, de stéaryle, d'isobornyle, de tertiobutyle; les acrylates de lauryle, de béhényle, de 2-éthylhexyle, de stéaryle, d'isobornyle, d'isobutyle; les macromonomères à base de poly (acrylate d'éthyl-2-hexyle) ou de poly(acrylate de dodécyle) à extrémité mono(méth)acrylate; des macromonomères éthylène/butylène et éthylène/propylène à groupement terminal réactif (méth)acrylate.

**21.** Polymère selon l'une des revendications 15 à 20, comprenant en outre au moins un monomère additionnel choisi parmi, seul ou en mélange, les monomères suivants :

- (i) les hydrocarbures éthyléniques ayant 2 à 10 carbones;
- (ii) les (méth)acrylates de formule $CH_2 = CHCOOR'_3$ ou $CH_2 = C(CH_3)COOR'_3$ dans lesquelles $R'_3$ représente :

  - un groupe alkyle linéaire ou ramifié, de 1 à 7 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle ou les atomes d'halogène (Cl, Br, I et F);
  - un groupe cycloalkyle en $C_3$ à $C_7$,
  - un groupe aryle en $C_3$ à $C_{20}$,

- un groupe aralkyle en $C_4$ à $C_{30}$ (groupe alkyle en $C_1$ à $C_8$),
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4),

lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle ou les atomes d'halogène,
- $R'_3$ peut également être un groupe $-(C_2H_4O)_m-R''$, avec m = 5 à 150 et R" = H ou alkyle de $C_1$ à $C_{30}$;

ou encore le méthacrylate d'isobutyle ou l'acrylate de tertiobutyle;
- (iii) les (méth)acrylamides de formule :

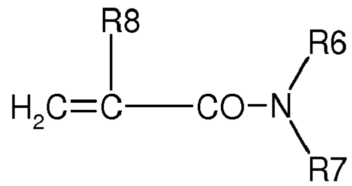

dans laquelle $R_8$ désigne H ou méthyle; et $R_7$ et $R_6$ identiques ou différents représentent :

- un atome d'hydrogène,
- un groupe alkyle linéaire ou ramifié, de 1 à 22 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F);
- un groupe cycloalkyle en $C_3$ à $C_{12}$,
- un groupe aryle en $C_3$ à $C_{20}$,
- un groupe aralkyle en $C_4$ à $C_{30}$ (groupe alkyle en $C_1$ à $C_8$),
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4),

lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F);
- (iv) les composés vinyliques de formules :

$$CH_2=CH-R_9,\ CH_2=CH-CH_2-R_9 \text{ ou } CH_2=C(CH_3)-CH_2-R_9$$

dans lesquelles $R_9$ est un groupe hydroxyle, halogène, $NH_2$, $OR_{14}$ où $R_{14}$ représente un groupe phényle ou un groupe alkyle en $C_1$ à $C_7$; acétamide ($NHCOCH_3$); un groupe $OCOR_{15}$ où $R_{15}$ représente un groupe alkyle de 2 à 7 carbones, linéaire ou ramifié; ou un groupe choisi parmi :

- un groupe alkyle linéaire ou ramifié, de 1 à 22 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F),
- un groupe cycloalkyle en $C_3$ à $C_{12}$,
- un groupe aryle en $C_3$ à $C_{20}$,
- un groupe aralkyle en $C_4$ à $C_{30}$ (groupe alkyle en $C_1$ à $C_8$),

- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4),

lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle les atomes d'halogène;
- (v) les monomères à insaturation(s) éthylénique(s) comprenant au moins une fonction acide carboxylique, phosphorique ou sulfonique, ou anhydride, comme par exemple l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,
- (vi) les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci.

**22.** Polymère selon la revendication 21, dans lequel les monomères additionnels sont choisis parmi, seul ou en mélange, l'acide (méth)acrylique et les (méth)acrylates d'alkyle en C1-C4, ou de cycloalkyle en C3-C7, et notamment le méthacrylate de méthyle, le méthacrylate d'isobutyle; l'acrylates de tertiobutyle, l'acide acrylique et l'acide méthacrylique.

**23.** Polymère selon l'une des revendications 15 à 22, dans lequel lesdites première et deuxième séquences sont reliées entre elles par une séquence (ou bloc) intermédiaire comprenant au moins un monomère constitutif de ladite première séquence et au moins un monomère constitutif de ladite deuxième séquence.

**24.** Procédé de préparation d'un polymère selon l'une des revendications 15 à 23, dans lequel ledit polymère est obtenu par polymérisation radicalaire en solution selon le procédé de préparation suivant :

- on peut introduire une partie du solvant de polymérisation dans un réacteur adapté, on chauffe jusqu'à atteindre la température adéquate pour la polymérisation (typiquement entre 60 et 120°C),
- une fois cette température atteinte, on peut ajouter les monomères constitutifs de la première séquence, en présence d'une partie de l'initiateur de polymérisation,
- au bout d'un temps T correspondant à un taux de conversion maximum de 90% de préférence, on peut introduire les monomères constitutifs de la deuxième séquence et l'autre partie de l'initiateur,
- on laisse réagir le mélange pendant un temps T' (allant notamment de 3 à 6 heures) au bout duquel le mélange est ramené à température ambiante (25°C), de manière à obtenir le polymère en solution dans le solvant de polymérisation.

Office européen des brevets

# RAPPORT DE RECHERCHE EUROPEENNE

Numéro de la demande
EP 07 12 0176

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | FR 2 867 681 A (OREAL [FR]) 23 septembre 2005 (2005-09-23) * revendications; exemple 2 * | 1-23 | INV. C08F293/00 C08F291/00 A61K8/90 |
| X | EP 1 690 526 A (OREAL [FR]) 16 août 2006 (2006-08-16) * revendications 11,26; exemples 1,2 * | 1-23 | |
| X | GU W ET AL: "THE GAS PERMEABILITY AND PACKING DENSITY OF COPOLYMERS OF METHACRYLOXYPROPYLTRIS (TRIMETHYLSILOXY) SILANE AND METHYLMETHACRYLATE" JOURNAL OF POLYMER SCIENCE, POLYMER PHYSICS EDITION, JOHN WILEY AND SONS. NEW YORK, US, vol. 29, no. 8, 1 juillet 1991 (1991-07-01), pages 1001-1007, XP000230189 ISSN: 0887-6266 * figures 1,2 * | 15-23 | |
| X | DATABASE COMPENDEX KAWAKITA HIROSHI; NAKAGAWA MASAO; NAGAI KAZUKIYO: ""Free radical polymerization and thermal properties of pendant adamantane copolymer"" XP002437179 Database accession no. E20064210181346 Polymer Preprints, Japan-55th SPSJ Annual Meeting May 24-26 2006; Vol 55 Nr. 1, Pg 475 * abrégé * | 15-23 | |
| | -/-- | | |

DOMAINES TECHNIQUES RECHERCHES (IPC)
C08F
A61K

Le présent rapport a été établi pour toutes les revendications

| Lieu de la recherche | Date d'achèvement de la recherche | Examinateur |
|---|---|---|
| Munich | 22 février 2008 | IRAEGUI RETOLAZA, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

4
EPO FORM 1503 03.82 (P04C02)

Office européen des brevets

# RAPPORT DE RECHERCHE EUROPEENNE

Numéro de la demande
EP 07 12 0176

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | G. HELARY, V. MIGONNEY, J. BELLENEY, L. HEINRICH: "Terpolymerization of 3-methacryloxypropyl tris(trimethylsiloxy)silane, methacrylic acid and dimethyl octyl ammonium styrene sulfonate: determination of the reactivity ratios" EUROPEAN POLYMER JOURNAL, vol. 30, 2000, pages 2365-2369, XP002437172 * figure 2 * ----- | 15-23 | |
| A,D | EP 1 411 069 A (OREAL [FR]) 21 avril 2004 (2004-04-21) * le document en entier * ----- | 1-23 | |
| A | DATABASE COMPENDEX août 2000 (2000-08), YOUNGBLOOD JEFFREY P; MCCARTHY THOMAS J: ""Nickel-mediated atom transfer radical polymeirzaiton (ATRP) of side-group siloxane containing block copolymers for controlled wettability"" XP002437180 Database accession no. E2000475364043 American Chemical Society, Polymer Preprints, Division of Polymer Chemistry. Vol 41 Nr. 2 Pg. 1554-1555 Conference Article. Washington, D.C. Meeting, Washington D.C., USA August 20-August 24 2000 * abrégé * ----- | 15-23 | DOMAINES TECHNIQUES RECHERCHES (IPC) |
| X | EP 1 518 534 A (OREAL [FR]) 30 mars 2005 (2005-03-30) * revendications 48,49; exemples * ----- | 24 | |
| X | US 2005/095213 A1 (BLIN XAVIER [FR] ET AL) 5 mai 2005 (2005-05-05) * revendications 70,78,128-130; exemples * ----- | 24 | |

Le présent rapport a été établi pour toutes les revendications

| Lieu de la recherche | Date d'achèvement de la recherche | Examinateur |
|---|---|---|
| Munich | 22 février 2008 | IRAEGUI RETOLAZA, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..................................................
& : membre de la même famille, document correspondant

4

EPO FORM 1503 03.82 (P04C02)

## ANNEXE AU RAPPORT DE RECHERCHE EUROPEENNE RELATIF A LA DEMANDE DE BREVET EUROPEEN NO. EP 07 12 0176

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

22-02-2008

| Document brevet cité au rapport de recherche | Date de publication | Membre(s) de la famille de brevet(s) | Date de publication |
|---|---|---|---|
| FR 2867681 A | 23-09-2005 | EP 1734919 A1 | 27-12-2006 |
| | | WO 2005092284 A1 | 06-10-2005 |
| | | JP 2007529482 T | 25-10-2007 |
| | | US 2007224158 A1 | 27-09-2007 |
| EP 1690526 A | 16-08-2006 | CN 1853611 A | 01-11-2006 |
| | | FR 2881950 A1 | 18-08-2006 |
| | | JP 2006225389 A | 31-08-2006 |
| EP 1411069 A | 21-04-2004 | BR 0303891 A | 08-09-2004 |
| | | CN 1504488 A | 16-06-2004 |
| | | JP 2004149772 A | 27-05-2004 |
| | | KR 20040027429 A | 01-04-2004 |
| | | MX PA03008714 A | 10-09-2004 |
| | | US 2004120920 A1 | 24-06-2004 |
| | | US 2008014158 A1 | 17-01-2008 |
| EP 1518534 A | 30-03-2005 | CN 1615811 A | 18-05-2005 |
| | | FR 2860142 A1 | 01-04-2005 |
| | | JP 2005126417 A | 19-05-2005 |
| | | KR 20050030614 A | 30-03-2005 |
| | | KR 20070048151 A | 08-05-2007 |
| US 2005095213 A1 | 05-05-2005 | AUCUN | |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1411069 A
- EP 895467 A
- EP 96459 A
- EP 1347013 A
- US 5625005 A


### Littérature non-brevet citée dans la description


- **BRANDRUP ; IMMERGUT ; GRULKE.** Polymer Handbook. John Wiley, 1999
- **GILLMAN.** *Polymer Letters,* 1967, vol. 5, 477-481